# EUROPÄISCHE PATENTSCHRIFT

(11) **EP 1 825 271 B1**
(45) Veröffentlichungstag und Bekanntmachung des Hinweises auf die Patenterteilung: **12.02.2014**
(21) Anmeldenummer: 05817949.0
(22) Anmeldetag: 01.12.2005
(51) Int. Cl.: G01N 33/566, G01N 33/68, C12Q 1/68

(54) **VERWENDUNG DER SERUM-/GLUCOCORTICOID REGULIERTEN KINASE**
USE OF A SERUM/GLUCOCORTICOID-REGULATED KINASE
UTILISATION D'UNE KINASE ACTIVEE PAR LE SERUM ET LES GLUCOCORTICOIDES

(30) Priorität: 10.12.2004 DE 102004059781
(43) Veröffentlichungstag der Anmeldung: 29.08.2007
(73) Patentinhaber: Sanofi-Aventis Deutschland GmbH, 65929 Frankfurt am Main (DE)
(72) Erfinder: BARTNIK, Eckart, 65205 Wiesbaden (DE); AIGNER, Thomas, Sanofi-Aventis Deutschland GmbH, 65926 Frankfurt am Main (DE); DIETZ, Uwe, Sanofi-Aventis Deutschland GmbH, 65926 Frankfurt (DE); BRIMMER, Annette, 50677 Köln (DE)
(86) Internationale Anmeldenummer: PCT/EP2005/012802
(87) Internationale Veröffentlichungsnummer: WO 2006/061130

(56) Entgegenhaltungen:
- WO-A-00/35946
- WO-A-00/73469
- WO-A-01/53312
- WO-A-01/53455
- WO-A-01/55440
- WO-A-02/24947
- WO-A-03/066835
- WO-A-03/083046
- WO-A-03/083117
- WO-A-2004/039956
- WO-A-2004/042022
- WO-A-2004/058153
- WO-A-2004/065577
- WO-A2-2004/069258
- US-A1- 2002 086 820

## Beschreibung

Die Erfindung betrifft die Verwendung der Serum/Glucocorticoid regulierten Kinase (SGK) zur Findung von Wirkstoffen. Weitere Aspekte der Erfindung beziehen sich auf Verfahren und Test Kits zur Identifizierung degenerativer Knorpelveränderungen.

Die durch Serum und Glucokortikoide induzierte Kinase SGK-1 wurde erstmals 1993 als "Immediate early Gene" in einer Ratten "Mammary"-Karzinom Zelllinie beschrieben (Webster et al., 1993a; Webster et al., 1993b). In diesen Zellen erfolgt als frühe Antwort auf die Gabe von Glukokortikoiden eine Induktion der SGK-1 Genaktivität und als Folge dessen eine signifikante Erhöhung der SGK-1 mRNA Mengen. In weiteren Arbeiten wurde gezeigt, dass SGK-1 und seine Indiuzierbarkeit sowohl in verschiedenen Zelllinien, als auch in Zellen von normalen Geweben auftritt (Brennan et al., 2000; Naray-Fejes-Toth et al., 2000; Cooper et al., 2001; Mikosz et al., 2001). SGK-1 gehört zu einer Familie von Serin/Threonin Kinasen, von der bislang drei Mitglieder bekannt sind und als SGK1, SGK-2 und SGK-3 bezeichnet werden. SGK-3 ist ausserdem unter den Namen SGKL (SGK-like) und CISK beschrieben. In ihrer katalytischen Domäne sind die drei Isoformen auf Proteinebene zu mindestens 80% untereinander homolog.

SGK-1 ist nahezu in allen Geweben exprimiert, die bislang getestet wurden, aber die Mengen an eprimierter mRNA variieren stark je nach Art des untersuchten Gewebetyps (Gonzalez-Robayna et al., 1999; Waldegger et al., 1999; Alliston et al., 2000; Klingel et al., 2000; Lang et al., 2000; Loffing et al., 2001; Fillon et al., 2002; Wamtges et al., 2002a;). Darüberhinaus wird SGK-1 mRNA in typischen embryonalen Geweben gefunden. Während der Mausembryogenese zeigt die SGK-1 mRNA Entwicklungs-dynamische Änderungen in spezifischen Geweben des Embryos (Decidua, Dottersack, Otic Vesicie) und ist während der Organogenese in Lungenknospen, Gehirn, Herz, Leber, Thymus etc. nachweisbar (Lee et al., 2001). SGK-2 ist mit grösster Abundanz in epithelialen Geweben exprimiert, so wie in der Niere, Leber, Pankreas und spezifischen Bereichen des Gehirns (Kobayashi et al., 1999b). SGK-3 konnte in allen bisher getesteten Geweben nachgewiesen werden und ist besonders im adulten Herzen und der Milz zu finden (Kobayashi et al., 1999b; Liu et al., 2000).

Einen Hinweis auf die Expression der SGK in degenerierendem oder degeneriertem Knorpelgewebe gab es bislang jedoch nicht.

Osteoarthrose (OA) ist eine der am häufigsten vorkommenden degenerativen Gelenkserkrankungen und führt im fortgeschrittenen Stadium zu einem Verlust der Gelenksfunktion. Während des chronischen Verlaufs der Krankheit kommt es zu einer Zerstörung des artikulären Knorpels bis auf das darunter liegende Knochengewebe, wodurch Gelenksersatzoperationen bei betroffenen Patienten notwendig werden. Neben der Zerstörung des Knorpels können auch pathologische Veränderungen an der Synovialmembran und den Ligamenten beobachtet werden. Die Krankheit wird wie die Rheumatoide Arthritis teilweise von entzündlichen Prozessen begleitet, unterscheidet sich jedoch von dieser.

Die genauen Ursachen der Erkrankung sind bislang nicht bekannt, es kommen jedoch verschiedene Faktoren in Frage, wie metabolische Veränderungen, mechanische Belastung, genetische Defekte oder Gelenksverletzungen.

Unabhängig vom ursprünglichen Auslöser tritt bei der OA als gemeinsames pathologisches Merkmal die Degradation des artikulären Knorpels ein. Ein Hauptmerkmal für den pathologischen Zustand der OA ist die proteolytische Spaltung von Kollagenen und Proteoglycanen. Gleichzeitig treten eine ganze Reihe weitere Prozesse wie anabole Reparaturmechanismen, Umdifferenzierung der Zellen oder Zelltod auf. Die genaueren molekularen Mechanismen dieser Prozesse sind noch weitgehend unverstanden.

Die gesunde Funktion des adulten Knorpels wird durch seine einzigartigen biomechanischen Eigenschaften geschaffen, die sowohl Widerstand gegen hohe Drucke als auch die notwendige Elastizität des Gewebes gewährleisten.-Entscheidend dafür ist die spezielle Organisation des Knorpelgewebes. Im Gegensatz zu den meisten anderen Geweben stehen die Zellen des Knorpels nicht in unmittelbarem Kontakt, sondern sind von einander getrennt in eine extrazelluläre Matrix (ECM) eingebettet. Die Makromoleküle dieser ECM garantieren die Funktionsfähigkeit des artikulären Knorpels und der Gelenke.

Das Grundgerüst der ECM besteht aus einem Netzwerk, das durch Fibrillen der Kollagene vom Typ II, IX und XI gebildet wird. Darin sind Proteoglykane eingelagert, hauptsächlich Aggrekan, wodurch eine extrem hohe osmotische Wasserbindungskapazität entsteht. Der dadurch erzeugte Schwelldruck in Verbindung mit den Eigenschaften des kollagenen Grundgerüsts garantieren die speziellen Eigenschaften des Knorpels.

Ein Hauptmerkmal der OA Pathogenese ist der Verlust der ECM des Knorpels und des artikulären Knorpelgewebes. Die Funktion des betroffenen Gelenks wird dadurch eingeschränkt oder geht verloren. Zusätzlich treten verschiedene symptomatische Parameter wie z.B. Schmerz während es Verlaufs der Krankheit auf.

Derzeitige Behandlungsmethoden der Osteoarthrose beschränken sich zumeist auf die Linderung symptomatischer Beschwerden. Eine kausale Therapie, die zum Rückgang der Knorpeldegeneration führt, ist auf medikamentöser Basis nach derzeitigem Erkenntnisstand nicht möglich. Insbesondere deshalb besteht erheblicher Bedarf an neuartigen Wirkstoffen zur Vorbeugung und/oder Behandlung der Osteoarthrose. Darüberhinaus besteht Bedarf an Möglichkeiten der frühzeitigen und sicheren Diagnose degenerativer Veränderungen des Gelenkknorpels, um möglichst im Anfangsstadium der Krankheit mit der Behandlung einsetzen zu können.

Die Aufgabe der Erfindung besteht demnach in der Bereitstellung neuartiger Möglichkeiten der Identifizierung von Wirkstoffen gegen degenerative Knorpelveränderungen.

Diese Aufgabe wird erfindungsgemäß gelöst durch die Verwendung eines SGK- A Proteins, eines funktionellen Derivats oder Fragments davon (auch funktioneller Derivate eines solchen Fragments) oder einer ein solches Protein, funktionelles Fragment oder Derivat kodierenden Nukleinsäure (sowie deren funktionelle, z.B. stabilisierte, Derivate) für die Findung von Wirkstoffen zur Vorbeugung oder Behandlung degenerativer Knorpelveränderungen, wie in den Ansprüchen beschrieben. Die Sequenz der humanen SGK Gene ist bekannt. Die kodierenden Polynukleotidsequenzen dieser Gene sind unter den Nummern NM_005627, AF153609 (SGK-1), AF169034, AF186470 (SGK-2), NM_013257, AF085233, AF169035 (SGK-3) bei der NCBI Nucleotide-Database abrufbar. Ebenso sind die Proteinsequenzen unter den Nummern NP_005618 (SGK-1), NP_733794. NP_057360 (SGK-2), Q96BR1 (SGK-3), bei der NCBI Protein Database zugänglich. Die Lokalisierung der humanen SGK Gene ist auf verschiedene Chromosomen verteilt. SGK-1 liegt auf Chromosom 6 (6q23), SGK-2 auf Chromosom 20 (20q13.2), SGK-3 auf Chromosom 8 (8q12.3-8q13.1). NCBI ist das National Center for Biotechnology Information (Postadresse: National Center for Biotechnology Information, National Library of Medicine, Building 38A, Bethesda, MD 20894, USA; Web-Adresse: www.ncbi.nhm.nih.gov). Die Klonierung des SGK-1 Gens wurde unter anderem beschrieben in Webster et al., 1993a; Waldegger et al, 1997; 1998; die Klonierung von SGK-2 und SGK-3 wurde erstmals durch Kobayashi et al., 1999b beschrieben.

Ein Unterscheidungsmerkmal von SGK zu vielen anderen Kinasen beruht auf der stringenten Stimulus-abhängigen Regulation der Transkription, der zellulären Lokalisation und der enzymatischen Aktivierung (Firestone et al., 2003) des Moleküls.

Zur Aktivierung der Transkription von SGK-1 kennt man eine Vielzahl von Stimuli. Dazu gehören Mineralkortikoide (Brennan et al., 2000; Shigaev et al., 2000; Bhargava et al., 2001), Gonadotropine (Richards et al., 1995; Gonzalez-Robayna et al., 2000), 1,25(OH)₂D₃ (Akutsu et al., 2001), p53, osmotische, Zellvolumen- und hypotonische Veränderungen (Waldegger et al., 1997; Klingel et al., 2000; Waldegger et al., 2000; Rozansky et al., 2002; Wamtges et al., 2002a), Zytokine wie GM-CSF und TNF-alpha (Cooper et al., 2001) oder durch TGF-beta (Kumar et al., 1999; Waldegger et al., 1999; Lang et al., 2000). Induktion von SGK erfolgt in weiteren Wachstums-abhängigen Signalwegen durch Serum (Webster et al., 1993a), Insulin und IGF-1 (Kobayashi et al., 1999a; Park et al., 1999; Perrotti et al., 2001) FSH (Alliston et al., 1997), Fibroblast-und Platelet-derived Growth Factor (Davies et al., 2000), Aktivatoren der Erk Signalkaskade (Hayashi et al., 2001) und TPA (Mizuno et al., 2001).

Es sind ausserdem einige pathologische Veränderungen bekannt, unter denen SGK-1 aktiviert wird, wie bei ischämischen Verletzungen des Gehirns (Imaizumi et al., 1994), viraler Hepatitis (Fillon et al., 2002), Lungenfibrose (Warntges et al., 2002b) oder Herzfibrose (Funder 2001).

Die Implikation von SGK in Prozesse degenerativer Gelenkserkrankungen war bislang jedoch nicht bekannt.

Gründsätzlich bewirken extrazelluläre Reize nicht generell eine Aktivierung von SGK-1. So kommt es z.B nach Gabe von Heparin zur Inhibition der SGK-1 Transkription in proliferierenden glatten Muskelzellen des vaskulären Systems (Delmolino et al., 1997).

Um in seine funktionsfähige Form umgewandelt zu werden, benötigt SGK-1 eine Aktivierung durch Phosphorylierung. Diese wird durch eine Signalkaskade vermittelt, an der Phosphatidylinositol (PI)-3 Kinase und die 3-Phosphoinositid abhängigen Kinasen PDK1 und PDK2 beteiligt sind.

Die Aktivierung von SGK-1 durch den PI-3 Kinase Signalweg ist als Antwort auf Insulin, IGF und Wachstumsfaktoren bekannt. Zur Aktivierung ist eine Phosphorylierung an zwei Aminosäureresten notwendig, Threonin²⁵⁶ am T-Loop und Serin⁴²² am hydrophoben Motiv des Proteins. Die Phosphorylierung an Threonin²⁵⁶ wird durch PDK1 vermittelt, die Phosphorylierung von Serin⁴²² soll duch eine putative PDK2 katalysiert werden, die bisher noch nicht bekannt ist (Kobayashi et al., 1999a; Park et al., 1999; Biondi et al., 2001).

Die Aktivierung durch PDK1 wurde an Hand von Mutationen der Phosphorylierungsstellen Threonin²⁵⁶ und Serin⁴²² gezeigt. Die Mutation von Threonin²⁵⁶ zu Alanin bewirkt eine Reduzierung der Phosphorylierung um 80-90% und verhindert eine PDK1 abhängige Aktivierung von SGK-1. Der gleiche Effekt wird beobachtet, wenn sowohl Threonin²⁵⁶ als auch Serin⁴²² zu Alanin mutiert werden. Dagegen bewirkt die Mutation von Serin⁴²² zu Aspartat eine ca. 6-fache Erhöhung der Phosphorylierung und Aktivierung durch PDK1 (Kobayashi et al., 1999a).

Die Funktion von SGK im Rahmen physiologischer oder pathologischer Prozesse ist derzeit noch nichtbekannt. Zur Funktion von SGK gibt es derzeit lediglich eine Reihe von Untersuchungen die zeigen, dass SGK-1,-2 und -3 regulatorischen Einfluss auf Kanäle der Zellmembran besitzen. So wurde gezeigt, dass der epitheliale Na⁺ Kanal (ENaC), der hauptsächliche Transporter für die Mineralkortikoid-regulierte Na⁺ Reabsorption im renalen Tubulus, ein Zielmolekül von SGK-1, SGK-2 und SGK-3 ist (Alvarez de la Rosa et al., 1999; Böhmer et al., 2000; Wagner et al., 2000a; Wang et al., 2001; Faletti et al., 2002; Friedrich et al., 2003). Die Interaktion von SGK mit ENaC erfolgt nicht durch direkte Phosphorylierung (Lang et al., 2000) sondern durch die Inaktivierung der Ubiquitin Ligase Nedd4-2 (Debonneville et al., 2001; Snyder et al., 2002) als Resultat der Phosphorylierung durch SGK. Dadurch erhöht sich die Menge und die Verweildauer von ENaC in der Zellmembran (Staub et al., 1997; Alvarez de la Rosa et al., 1999; Wagner et al., 2000a). Des weiteren wurde in einer Reihe von Experimenten gezeigt, dass ROMK1 ein Zielmolekül von SGK ist. Allerdings wird ROMK1 nicht durch SGK direkt reguliert, sondern benötigt dazu als Vermittlermolekül den "Na⁺/H⁺ exchange regulating factor 2" (NHERF2) (Shenolikar et al., 2001; Yun et al., 2003).Das gleiche gilt für ein weiteres Zielmolekül von SGK, den Na⁺/H⁺ Transporter NHE3 (Yun et al., 2002).Zudem wurde in Experimenten an Xenopus Oozyten gezeigt, dass SGK den vom Kv1.3 Kanal abhängigen K⁺ Strom beeinflusst (Gamper et al., 2002b; Wamtges et al., 2002a). Ausserdem wurde beschrieben, dass SGK die Aminosäure Transporter 42F/LAT und SN1 reguliert ((Wagner et al., 2000b; Böhmer et al., 2003a,b).

Ein Bild der SGK Funktion im Rahmen physiologischer oder pathologischer Prozesse war jedoch trotz dieser Befunde bislang nur teilweiserekonstruierbar.

Fragmente der SGK sind Polypeptide oder Oligopeptide (bzw. diese kodierende Polyoder Oligonukleotide), die sich durch Weglassen einer oder mehrerer Aminosäuren von den Enden oder aus dem Inneren der natürlich vorkommenden SGK, vorzugsweise der humanen SGK und besonders bevorzugt der SGK nach einer der Sequenzen gemäß SEQ ID No. 1, 2 oder 3, unterscheiden. Bevorzugte Beispiele funktioneller Fragmente enthalten oder bestehen aus der SGK Kinasedomäne (s. Kobayashi et al., 1999a). Besonders bevorzugt sind hierbei die Fragmente, welche Sequenzen gemäß SEQ ID No. 7-12 umfassen und insbesondere daraus bestehen. Derivate der SGK sind Polypeptide oder Oligopeptide mit SGK-Funktion, die sich durch Austausch mindestens einer Aminosäure und/oder durch Zufügung mindestens einer Aminosäure und/oder chemischer Modifikation mindestens einer Aminosäure, wie z.B. der Biotinylierung, Phosphorylierung etc. von dem natürlich vorkommenden SGK Protein, vorzugsweise der humanen SGK und insbesondere der SGK nach einer der Sequenzen gemäß SEQ ID No. 1, 2 oder 3 unterscheiden.

Funktionelle Fragmente oder Derivate der SGK sind Poly- oder Oligopeptide oder diese kodierende Poly- oder Oligonukleotide, die mindestens eine Funktion der SGK besitzen. Diverse Funktionen der SGK sind nachfolgend beschrieben, z.B. die Regulation des Zellvolumens (Fillon et al., 2001), die Aktivierung epithelialer Na-Kanäle (EnaC), (Debonneville et al., 2001); die Aktivierung des Na+-Transportes (Rozansky et al., 2002 oder Lang and Cohen, 2001) oder des Aminosäuretransportes (Wagner et al., 2000), oder die Regulation des KCNE1 abhängigen K+-Flusses; die Kinaseaktivität der SGK, z.B. die Phosphorylierung der Ubiquitin Ligase P oder die Phosphorylierung von Nedd4-2 an Serin 444 und/oder Serin 338 (Snyder et. al., 2002); die Fähigkeit, der Autophosphorylierung oder die Fähigkeit, bestimmte Peptide zu phosphorylieren (s. z.B. Kobayashi et al., 1999a). Die Fähigkeit mit bestimmten Proteinen zu interagieren, z.B. einem der vorher genannten, gehört ebenso zu den Funktionen der SGK. Besonders gut eignet sich hierbei die Fähigkeit der SGK, Ser und Thr Reste in Arg-Xaa-Arg-Xaa-Xaa-Ser/Thr Motiven (vgl. Kobayashi et al., 1999a), insbesondere in einem Oligo- oder Polypeptid welches die folgende Sequenz enthält oder daraus besteht zu phosphorylieren: GRPRTSSFAEG. Die Fragmente oder Derivate eines bestimmten SGK Subtyps können dabei mindestens eine Funktion aufweisen, die allen SGK Subtypen gemeinsam ist (z.B. die Fähigkeit der SGK, Ser und Thr Reste in Arg-Xaa-Arg-Xaa-Xaa-Ser/Thr Motiven bzw. Peptide der Sequenz GRPRTSSFAEG zu phosphorylieren). Vorzugsweise weisen sie eine Funktion auf, die spezifisch für den jeweiligen Subtyp ist (so z.B. die unterschiedliche Aktivität bei der Phosphorylierung bestimmter synthetischer Peptide unterschiedlicher Sequenz, s. z.B. Kobayashi et al. 1999a).

Test-Methoden zur Feststellung der Proteinaktivität sind dem zuständigen Fachmann hinlänglich bekannt. So kann mittels herkömmlicher Methoden z.B. die Phosphorylierungsaktivität, die Interaktion mit bestimmten Zielproteinen oder Fragmenten davon, oder die Fähigkeit der SGK bestimmt werden, die Aktivität bestimmter Ionenkanäle und Transporter zu modulieren (s. hierzu auch unten). Gemäß einer zweckmäßigen Ausführungsform wird SGK dazu als isoliertes Molekül eingesetzt.

Im Rahmen der vorliegenden Erfindung bedeutet der Terminus "isoliert" in Bezug auf Polypeptide / Proteine / Polynukleotide / Nukleinsäuren und deren Fragmente und Derivate, dass diese Moleküle sowohl aus natürlichen Quellen aufgereinigt, als auch rekombinant hergestellt und aufgereinigt worden sein können (wobei der Begriff "aufgereinigt" auch "teilweise aufgereinigt" umfasst).

Die Herstellung isolierter SGK Moleküle ist dem zuständigen Fachmann hinlänglich bekannt. So können anhand der bekannten genomischen oder kodierenden Polynukleotidsequenzen mittels der Polymerase Kettenreaktion Polynukleotide gewünschter Länge amplifiziert und anschliessend mittels Klonierung in Wirtszellen vervielfacht werden (s. hierzu u.a. die unten aufgeführte Standardliteratur). Die Polymerase Ketten Reaktion (PCR) ist eine in vitro Technik, mit der Polynukleotid Abschnitte, die von zwei bekannten Sequenzen eingerahmt werden, gezielt vervielfältigt werden können. Dabei reicht es aus, wenn die 5' von der zu amplifizierenden Sequenz liegende Sequenz bekannt ist. In diesem Fall kann mittels herkömmlicher Techniken (z.B. Restriktionsverdau, etc.) ein Fragment des zu amplifizierenden Fragmentes generiert werden, an dessen 3'-Ende dann ein DNA-Molekül bekannter Sequenz ligiert wird (sogenannter "Linker"), so dass die zu amplifizierende Sequenz schliesslich von zwei bekannten Sequenzen umrahmt wird (sogenannte Linker-vermittelte PCR, ImPCR).

Zur Amplifikation werden kurze, einzelsträngige DNA-Moleküle verwendet (Primer), die komplementär zu den Sequenzbereichen der DNA oder RNA Matrize sind die das zu amplifizierende Polynukleotid einrahmen. Unter definierten Reaktionsbedingungen werden die Primer in Gegenwart von Desoxynukleotidtriphosphaten (dNTPs) entlang der einzelsträngigen, denaturierten Matrize durch bestimmte Enzyme, sogenannte Polymerasen (DNA Polymerasen, die DNA als Matrize erkennen und DNA Stränge hervorbringen, oder Reverse Transkriptasen, die RNA als Matrize erkennen und DNA Stränge hervorbringen) verlängert, so dass neue DNA Stränge entstehen, deren Sequenz komplementär zu der der Matrize ist. Durch die Wahl bestimmter Temperaturabfolgen, die sich periodisch wiederholen, wird sichergestellt, dass die neu generierten Polynukleotidstränge denaturiert werden, anschliessend Primer daran hybridisieren und schliesslich eine neue Verlängerunsreaktion stattfindet, usw. Um die für die Denatuierung notwendigen Temperaturen einsetzen zu können, ohne neues Enzym nachzufügen, werden zweckmäßigerweise hitzestabile Polymerasen eingesetzt, z.B. Taq-Polymerase (DNA Polymerase aus Thermus aquaticus). Die Auswahl der geeigneten Polymerase hängt von dem Einsatzzweck ab und ist dem zuständigen Fachmann bekannt. So ist für den Einsatz der PCR zum Zwecke der Klonierung beispielsweise die Wahl von Polymerasen mit der Fähigkeit zur Fehlerkorrektur (sog. Proofreading) vorteilhaft.

Ein typischer PCR Ansatz enthält neben einer Polynukleotid Matrize zwei geeignete Primer, beispielsweise in Konzentrationen zwischen 0,2 bis 2µM, weiterhin dNTPs, beispielsweise mit Konzentrationen von 200µM pro dNTP, weiterhin MgCl₂ mit einer Konzentration von 1-2 mM und 1-10 Einheiten einer hitzestabilen DNA-Polymerase wie beispielsweise Taq, sowie beispielsweise 0,01 bis 20 ng Polynukleotid (also DNA oder RNA) Matrize.

Die Generierung von Primern oder Oligonukleotiden erfolgt durch chemische Synthese nach herkömmlichen Protokollen. Die Synthese wird dabei herkömmlicherweise von kommerziellen Anbietern, wie MWG Biotech u.a. vorgenommen. cDNA verschiedensten Ursprunges ist kommerziell erhältlich, so z.B. von Anbietern wie Promega, Stratagene, etc. Geeignete Puffer und Enzyme zur Durchführung der PCR sind dem zuständigen Fachmann ebenfalls bekannt und auch kommerziell erhältlich. Eine weitere Methode der Generierung isoliert vorliegender Polynukleotidsequenzen besteht in der Klonierung der gewünschten Sequenzen mittels bekannter Methoden, ihrer anschliessenden Expression in geeigneten Organismen, vorzugsweise Einzellern wie beispielsweise geeigneten Bakterien- oder Hefestämmen und der anschliessenden (zumindest partiellen) Aufreinigung des exprimierten Polynukleotids.

Zur Herstellung eines zu klonierenden Polynukleotidfragmentes eignet sich beispielsweise die PCR Reaktion. Vorteilhafterweise wird dabei zumindest ein Primer verwendet, welcher in seinem 5'-Anteil die Erkennungssequenz einer Restriktionsendonuklease (sogenannte Restriktionsschnittstelle) trägt. Vorteilhafterweise weisen beide Primer eine solche Schnittstelle auf, wobei beide Schnittstellen gleich oder verschieden gewählt werden können. Gängige Restriktionsenzyme sind z.B: BamHI (GGATCC), Clal (ATCGAT), EcoRI (GAATTC), EcoRV (GATATC), HindIII (AAGCTT), Ncol (CCATGG), Sall (GTCGAC), Xbal (TCTAGA), etc. Die Auswahl geeigneter Restriktionsenzyme und geeignete Reaktionsbedingungen sind dem zuständigen Fachmann hinlänglich bekannt.

Zur Klonierung wird die DNA eine Vektors durch geeignete Restriktionsendonukleasen geschnitten ("verdaut"), die auch entsprechende Erkennungsstellen in den Primersequenzen des zu klonierenden cDNA Fragments besitzen. Das beispielsweise mittels PCR generierte Fragment wird durch Isolierung und Behandlung mit den selben Restriktionsenzymen kompatibel zum "verdauten" Vektor gemacht. Anschliessend erfolgt nach Aufreinigung von Vektor und behandeltem Fragment die Zusammenfügung mittels einer DNA-Ligase bei geeigneten Reaktionsbedingungen (diese sind dem Fachmann bekannt).

Ein Vektor ist ein zirkuläres oder lineares DNA Molekül, etwa ein Plasmid, Bakteriophage oder Cosmid, mit dessen Hilfe DNA Fragmente in geeigneten Organismen gezielt amplifiziert werden können (sog. Klonierung). Geeignete Organismen sind zumeist Einzeller mit hohen Wachstumsraten, wie z.B. Bakterien oder Hefen, können jedoch auch Zellen aus vielzelligen Verbänden sein, wie z.B. aus verschiedensten Organismen generierte Zelllinien (z.B. SF9 Zellen aus Spodoptera Frugiperda, etc.). Geeignete Vektoren sind dem Fachmann hinlänglich bekannt und bei Biotechnologie Zulieferern wie z.B. Roche Diagnostics, New England Bioloabs, Promega, Stratagene, u.v.m kommerziell erhältlich.

Mittels geeigneter spezieller Vektoren, die ebenfalls im Stande der Technik bekannt sind, kann anhand der klonierten Polynukleotide auch die rekombinante Herstellung isolierter Polypeptide erfolgen. Dies kann durch Expression in geeigneten Wirtszellen, z.B. bakteriellen (vorzugsweise E. coli Stämme) oder eukaryontischen Wirten (z.B. SF9 Zellen, etc.) erfolgen, wobei die Polynukleotide jeweils in spezielle Expressionsvektoren kloniert und anschliessend in die entsprechenden Zellen eingeschleust werden. Geeignete Transformations- bzw. Transfektionsmethoden sind dem Fachmann ebenfalls bekannt (s. z.B. angefügte Standardliteratur), ebenso die Bedingungen der Zellkultivierung und Induktion der Proteinexpression. Eine andere Möglichkeit, isolierte Polypeptide rekombinant herzustellen besteht in der in vitro Translation. Auch hierzu wird das Polynukleotid in einen geeigneten Vektor kloniert und anschliessend in geeigneten Puffern und Zellextrakten (z.B. in Retikulozytenlysaten) das Polypeptid in vitro exprimiert. Vektoren, weitere notwendige Reagenzien und Bedingungen sind ebenfalls hinlänglich bekannt und kommerziell (z.B. durch Invitrogen) erhältlich.

Im Stande der Technik sind drei SGK Subtypen (SGK 1 bis 3) bekannt. Beansprucht wird die Verwendung von SGK 1, im Rahmen der verschiedenen Aspekte der vorliegenden Erfindung , z.B. der erfindungsgemäße Nachweis der Expression von SGK 1 (siche Ansprüche).

Die Erfindung beruht auf Ergebnissen der Erfinder, die anhand vergleichender Genexpressionsanalysen von Proben gesamt-zellulärer RNA aus gesundem und degeneriertem/degenerierendem Knorpelgewebe überraschend gezeigt haben, dass die Serum/Glucocorticoid Regulierte Kinase-1 (SGK-1) in degeneriertem/degenerierendem osteoarthrotischen Knorpel exprimiert wird, während sie in gesundem artikulären Knorpelgewebe nicht nachweisbar ist. Es handelt sich hierbei um den erstmaligen Nachweis des Moleküls in Knorpelgewebe. Darüberhinaus wurden durch weitere Experimente der Erfinder erstmals Hinweise auf die kausale Implikation von SGK bei der Entstehung degenerativer Knorpelveränderungen geliefert.

Die Expression von SGK-1 im Knorpelgewebe stellt dabei einen völlig neuen Befund dar, da im Stand der Technik keinerlei publizierte Untersuchungen zur Rolle von SGK in diesem Gewebe oder zu möglichen regulatorischen Mechanismen der Expression von SGK unter pathologischen und entwicklungsspezifischen Parametern in Knorpelzellen bekannt sind. Anhand der Studien der Erfinder läßt sich erstmals folgern, dass SGK spezifisch mit pathologischen Zuständen des Knorpels, so zum Beispiel im Rahmen der Rheumatoiden Arthritis oder der Osteoarthrose, insbesondere jedoch im Rahmen der Osteoarthrose verknüpft ist, und somit ein Schlüsselmolekül zur Auslösung Knorpel-degradativer Prozesse darstellt.

Auf Grund der hohen Homologie zwischen den SGK Familienmitgliedern, ist davon auszugehen, dass dies für die SGK-2 und SGK-3 ebenso gilt. (nicht beansprucht). Die erstmalige Identifizierung dieser Zusammenhänge ermöglicht die Findung von Wirkstoffen zur Vorbeugung oder Behandlung degenerativer Knorpelveränderungen, indem durch bekannte Testmethoden die Wirkung potentieller Wirkstoffe auf die Aktivität von SGK und/oder den Spiegel an SGK festgestellt wird. Die kausale Implikation der SGK bei der Entstehung degenerativer Gelenkserkrankungen ermöglicht dabei die gezielte Suche nach therapeutischen Wirkstoffen, die auf regulative Mechanismen zur Wiederherstellung der normalen Zellphysiologie des Knorpels abzielen.

Ein weiterer Aspekt der Erfindung bezieht sich demnach auf ein Verfahren zur Identifizierung von Wirkstoffen zur Vorbeugung oder Behandlung degenerativer Knorpelveränderungen mit den Schritten
a. Kontaktierung eines SGK Proteins, eines funktionelles Derivats oder Fragments davon, mit einem potentiellen Wirkstoff.
b. Bestimmung der SGK Aktivität in Anwesenheit des potentiellen Wirkstoffs.
c. Bestimmung der SGK Aktivität in Abwesenheit des potentiellen Wirkstoffs.
d. Vergleich der SGK Aktivität nach b mit der nach c); wobei vorzugsweise bestimmt wird, ob die Aktivität der SGK (Protein, funktionellem Derivat oder Fragment davon) in Anwesenheit des potentiellen Wirkstoffs geringer ist als in dessen Abwesenheit, sich also aktive Wirkstoffe vorzugsweise anhand ihrer Fähigkeit auszeichnen, die Aktivität der SGK zu inhibieren.

Ein weiterer Aspekt der Erfindung bezieht sich auf ein Verfahren zur Identifizierung von Wirkstoffen zur Vorbeugung oder Behandlung degenerativer Knorpelveränderungen mit den Schritten
a) Kontaktierung einer Probe, die ein SGK Protein, ein funktionelles Derivat oder Fragment einer dafür kodierenden Nukleinsäure enthält, mit einem potentiellen Wirkstoff in einem System, das zumindest zur Transkription fähig ist.
b) Bestimmung der Menge an SGK Protein, funktionellem Derivat oder Fragment davon oder der diese kodierende mRNA in Anwesenheit des potentiellen Wirkstoffs.
c) Bestimmung der Menge an SGK Protein, funktionellem Derivat oder Fragment davon oder der diese kodierende mRNA in Abwesenheit des potentiellen Wirkstoffs.
d) Vergleich der Protein oder mRNA Mengen nach b) und nach c), wobei vorzugsweise bestimmt wird, ob Menge an SGK (Protein, funktionellem Derivat oder Fragment davon oder die der diese kodierenden mRNA) in Anwesenheit des potentiellen Wirkstoffs geringer ist als in dessen Abwesenheit, sich also aktive Wirkstoffe vorzugsweise anhand ihrer Fähigkeit auszeichnen, die Menge zur Verfügung stehender SGK zu mindern.

Des Weiteren bezieht sich die Erfindung auf ein Verfahren zur Identifizierung von Wirkstoffen zur Vorbeugung oder Behandlung degenerativer Knorpelveränderungen mit den Schritten
a) Kontaktierung einer Probe, die ein SGK Protein, ein funktionelles Derivat oder Fragment davon, oder eine dafür kodierende Nukleinsäure enthält, mit einem potentiellen Wirkstoff in einem System, das zumindest zur Translation fähig ist.
b) Bestimmung der Aktivität des SGK Proteins, Fragments oder Derivats in Anwesenheit des potentiellen Wirkstoffs.
c) Bestimmung der Aktivität des SGK Proteins, Fragments oder Derivats in Abwesenheit des potentiellen Wirkstoffs, wobei vorzugsweise bestimmt wird, ob die SGK Aktivität oder Menge (Protein, funktionellem Derivat oder Fragment davon oder die der diese kodierenden mRNA) in Anwesenheit des potentiellen Wirkstoffs geringer ist als in dessen Abwesenheit, sich also aktive Wirkstoffe vorzugsweise anhand ihrer Fähigkeit auszeichnen, die Aktivität der SGK zu inhibieren, oder die Menge zur Verfügung stehender SGK zu mindern.

Die Feststellung, ob die SGK Aktivität in Anwesenheit des potentiellen Wirkstoffs geringer ist als in dessen Abwesenheit kann z.B. an Hand der Fähigkeit bestimmt werden, synthetische Peptide oder andere SGK Substrate zu phosphorylieren. (Darunter fällt auch die Autophosphorylierung.) Bevorzugt ist hierbei die Verwendung von Poly- oder Oligopeptiden, die das Sequenzmotiv Arg-Xaa-Arg-Xaa-Xaa-Ser/Thr, vorzugsweise das Motiv RPRTSS und insbesondere das Motiv GRPRTSSFAEG enthalten oder daraus bestehen. Eine weitere Möglichkeit besteht in der Bestimmung der Fähigkeit der SGK, die Aktivität bestimmter Ionenkanäle zu modulieren; diese ist z.B. messbar anhand intrazellulärer Ionenkonzentrationen oder anderer dem zuständigen Fachmann bekannter Methoden.

Die Aktivität der SGK bezieht sich dabei auf alle Funktionen der SGK-1 (s. auch oben). Diese können durch die potentiellen Wirkstoffe auf allen Ebenen moduliert werden, die sich auf die Proteinaktivität ausüben (z.B. durch direkte Interaktion mit der Kinasedomäne; Beeinflussung der posttranslationalen Modifikation, die sich auf die Protein Aktivität auswirken kann, Beeinflussung der Protein Faltung oder der Aktivierung durch natürliche Inhibitoren oder Aktivatoren der SGK, wie z.B. PI3K).

Die SGK Menge bezieht sich auf das Gleichgewicht aktiver SGK in einem System, z. B. in einer Zelle oder einem biochemischen Ansatz zu einem bestimmten Zeitpunkt. Die SGK Menge kann dabei auf allen Ebenen der SGK Expression oder des SGK Abbaus moduliert werden (Transkription einschl. Transkript-Prozessierung und - Ausschleusung aus dem Zellkern oder Translation oder der posttranslationalen Modifikation, welche sich auf die Protein Stabilität und somit auf das Gleichgewicht aktiver zur Verfügung stehender SGK auswirken kann).

Wirkstoffe, die die Fähigkeit haben, SGK Aktivität und/oder die SGK Menge zu modulieren, vorzugsweise zu mindern, sollten dabei aufgrund der funktionellen Implikation von SGK bei der Veränderung von Knorpelzellen zu einem degenerativen Status, die Entstehung von degenerativen Knorpelveränderungen hemmen und vorzugsweise unterbinden und / oder die Auswirkungen bestehender Knorpelveränderungen mindern und vorzugsweise aufheben.

Geeignete analytische Methoden oder Systeme, sogenannte Assays, die die Aktivität oder die Konzentration bzw. Menge bestimmter Zielmoleküle (hier der SGK) des Körpers (sogenannte "Targets", in der Regel Proteine oder Nukleinsäuren), als Parameter der Wirksamkeit potentieller Wirkstoffe messen, sind im Stande der Technik bekannt. Diese können beispielsweise in vitro Assays sein, also biochemische Assays mit isolierten oder teil-isolierten Komponenten, die zu einem Reaktionsgemisch zusammengesetzt werden, anhand dessen sich die Wirksamkeit potentieller Wirkstoffe messen lässt. Darüber hinaus können dies auch zelluläre Testsysteme (Assays) sein, in denen die Aktivität des Zielproteins (hier SGK) und die Wirksamkeit potentieller Wirkstoffe auf die Aktivität dieses Zielmoleküs im zellulären Umfeld bestimmt werden kann.

Ein Assay ist dabei jede Art analytischer Methode, anhand derer ein biologischer Prozess überwacht werden kann. Dabei werden herkömmlicherweise molekulare Abläufe und Signalkaskaden, die Teile physiologischer Stoffwechselwege und Regelmechanismen, aber auch pathologischer Zustände darstellen in zellulären oder biochemischen Systemen nachgestellt. Die pharmakologische Aktivität eines Wirkstoffes kann dann anhand seiner Fähigkeit, in diese Wege und Mechanismen einzugreifen, bestimmt werden.

Zum Einsatz im Rahmen der Wirkstoff-Findung, insbesondere des Hochdurchsatzscreenings nach Wirkstoffen, muss der Assay reproduzierbar sein und ist vorzugsweise auch skalierbar und robust (also wenig anfällig gegen äussere Einflüsse). Der Assay sollte vorzugsweise für das Hochdurchsatzscreening chemischer Substanzen nach ihrer Fähigkeit, sich auf die Aktivität von Zielmolekülen auszuwirken, geeignet sein. Die Art des Assays hängt dabei unter anderem von der Art des verwendeten Zielmoleküls (z.B. genauer Typ oder Art von biochemischem Grundmolekül, z.B. Polypeptid oder Polynukleotid) und dem "read out", d.h. die Parameter, anhand der die Aktivität des Zielmoleküls bestimmt wird, ab.

Im Stand der Technik sind verschiedene Assaytypen bekannt und großteils auch kommerziell von gewerblichen Anbietern erhältlich.

Zur Messung der Interaktion zweier Bindungspartner geeignete Assays enthalten z.B. radioisotopische oder fluroreszierende Assays, z.B. Fluoreszenzpolarisierungsassays, wie z.B. kommerziell von Panvera, Perkin-Elmer Life Sciences (NEN™, LANCE™) oder Packard BioScience (HTRF; ALPHAscreen™) erhältlich. Die vorgenannten Assays eignen sich zur Messung der Interaktion einer markierten Komponente mit einer nicht markierten Komponente (z.B. markierte Proteine und deren Interaktion mit nicht-markierten Liganden).

Weitere Beispiele von Assays enthalten zelluläre Assays, in denen eine Zelllinie stabil (induzierbar oder konstitutiv; chromosomal oder episomal) oder transient ein rekombinantes Protein nach Wunsch exprimiert. Diese Assays enthalten z.B. Reportergen Assays, in denen die Regulation eines bestimmten Promotors oder die Regulation eines Signaltransduktionsweges oder eines Mitgliedes einer Signaltransduktionskaskade anhand der Aktivität eines Reporterenzyms gemessen wird, dessen Expression unter der Kontrolle des betreffenden Promotors steht. Für diese Art von Assay ist es notwendig, eine rekombinante Zelllinie zu generieren, die das Reportergen unter der Kontrolle eines definierten Promotors exprimiert, der selbst zur Untersuchung steht, oder der durch die zu untersuchende Signaltransduktionskaskade reguliert wird. Geeignete Reporterenzyme sind dem zuständigen Fachmann allgemein bekannt und enthalten Feuerfliegen Luziferase, Renilla Luziferase (beide kommerziell z.B. durch Packard Reagents erhältlich), ß-Galaktosidase, etc. Die Auswahl geeigneter Zelllinien ist dem zuständigen Fachmann bekannt und hängt u. a. vom Ziel des Assays oder dem "read out" ab. In der Regel sind dies Zelllinien, die einfach zu kultivieren und zu transfizieren sind, so wie z.B. HeLA, COS, CHO oder NIH-3T3 Zellen.

Assays zur Messung der intrazellulären Ionenkonzentration beinhalten z.B. sogenannte FLIPR™ (fluorometric imaging plate reader, kommerziell erhältlich bei Molecular Devices) -Assays, in denen eine Argonlaser Lichtquelle in Kombination mit einer gekühlten CCD Kamera die gleichzeitige Messung der intrazellulären Ionenkonzentration (z.B. Na⁺ oder Ca²⁺, etc.) unterschiedlicher Ansätze in Zellen (z. B. Zellen, die rekombinant oder natürlich bestimmte Ionenkanäle exprimieren) erlaubt, die auf 384 well Platten kultiviert werden. Anhand dieser FLIPR™ Assays kann z.B. der intrazelluläre Kalziumspiegel mittels bestimmter Fluorochrome, z.B. Fluo-3, Fluo-4, der intrazelluläre pH unter Verwendung von BCECF, BCPCF oder spezifischer FLIPR™ Assay Kits, Änderungen des Membranpotentials unter Verwendung von z.B. DiBAC oder spezieller FLIPR™ Assay Kits oder Veränderungen der Membranpolarisation gemessen werden. Zur Bestimmung der Konzentration anderer Ionen wie Zink, Natrium, etc. sind andere Farbstoffe geeignet, die ebenfalls im Stande der Technik bekannt und kommerziell erhältlich sind.

Zur Bestimmung der Aktivität bestimmter Ionenkanäle, (die z.B. intrazelluläre Ionenkonzentrationen kontrollieren und deren Aktivität so anhand der intrazellulären Konzentration bestimmter Ionen determiniert werden kann) eignen sich z.B. Assays und Farbstoffe, die sensibel auf Veränderungen des Membranpotentials reagieren, z.B. DiBAC oder der Membran Potential Assay Kit für den FLIPR™ Assay von Molecular Devices, die Messung der mitochondrialen Membranpolarisation durch JC-1 Farbstoff mit der FLIPR™ Technologie; Ionen-sensitive Farbstoffe wie Fluo-3, Fluo-4 oder der Kalzium Assay Kit von Molecular Devices mit der FLIPR™ Technologie zur Bestimmung intrazellulärer Ionenkonzentrationen; Natriumsensitive Farbstoffe, z.B. von Molecular Probes zur Bestimmung der intrazellulären Natriumkonzentration; zur Bestimmung der intrazellulären Kaliumkonzentration z.B. Assays basierend auf dem Patch Clamping oder auf der Rubidium Ionen Efflux Messung anhand atomarer Adsorptionsspektroskopie, etc. Weitere automatische Vorrichtungen und Roboter zur Messung und Bestimmung bestimmter Veränderungen und Zustände in Zellen sind dem zuständigen Fachmann bekannt und enthalten z.B. den Acumen™ Detector von Acumen Bioscience, eine Fluroeszenz basierte Laser Scanning Lesevorrichtung, die die dreidimensionale Rekonstitution der Verteilung geeignet markierter Objekte ermöglicht.

Zur Messung der Proteinphosphorylierung bzw. der Kinaseaktivität eignen sich z.B. die Fluoreszenzpolarisierung, z.B. kommerziell erhältlich durch Panvera, Homogeneous Time Resolved Fluorescence (HTRF, Cis Bio) oder LANCE™ Assays (Perkin elmer Life Sciences) oder der Amplified Luminescent Proximity Homogeneous Assay (ALPHAScreen™ von Packard BioScience).

Andere Arten von Assays und andere Arten des "read outs" sind dem Zuständigen Fachmann ebenfalls hinlänglich bekannt

Ein weiterer Aspekt der Erfindung bezieht sich auf einen Hochdurchsatzscreen zur Wirkstoffindung basierend auf einem der vorgenannten Verfahren. Hochdurchsatzscreen bedeutet im Rahmen dieser Erfindung, dass das Verfahren in Kleinstmaßstäben, beispielsweise auf 96, 386 oder 1536 well Platten bzw. in Ansätzen mit geringen Volumina im Bereich von wenigen Millilitern bis zu wenigen Nanolitem durchgeführt wird, so dass binnen kürzester Zeit eine Vielzahl von Ansätzen, z.B. 500000 oder mehr, analysiert werden können.

Die vorgenannten erfindungsgemäßen Verwendungen und Verfahren, sowie der vorgenannte Hochdurchsatzscreen dienen der Findung von Wirkstoffen zur Behandlung und/oder Vorbeugung degenerativer Knorpelveränderungen. Insbesondere dienen sie der Findung von Wirkstoffen, die die Fähigkeit von SGK derartige degenerative Prozesse hervorzurufen und zu beschleunigen, inhibieren können. Inhibitorisch bedeutet im Rahmen der verschiedenen Aspekte dieser Erfindung, geeignet, die Funktion der SGK1 zu inhibieren. Diese inhibition kann auf verschiedensten Mechanismen beruhen, beispielsweise durch Senkung des zellulären SGK Spiegels (z.B. durch Verminderung der Transkription und/oder Translation, Senkung der Stabilität von Protein oder mRNA, etc.), Inhibierung der enzymatischen Aktivität der SGK oder negative Beeinflussung vor- oder nachgeschalteter Signalwege.

Bevorzugt ist hierbei die Senkung des zellulären SGK Spiegels oder Inhibierung der enzymatischen Aktivität.

Darüber hinaus bezieht sich die Erfindung auf ein Verfahren zur Identifizierung entstehender und bestehender degenerativer Knorpelveränderungen, welches den Nachweis von von SGK-1 im Knorpelgewebe enthält.

Der SGK Nachweis erfolgt dabei durch dem Fachmann bekannte Methoden. So kann anhand geeigneter Methoden, wie etwa dem Nachweis der SGK im degenerierten oder degenerierenden Knorpel auf Basis von immunohistochemischen Nachweisen von SGK Protein oder RNA (beispielsweise der immunohistochemischen Anfärbung histologischer Schnitte von Gewebeproben, Immunoblots, Elisas oder Protein Microarrays) oder durch Nachweis der SGK RNA mittels Northernblottings, quantitativer PCR, in situ Hybridisierungen oder DNA Microarrays, etc. das Vorhandensein von SGK im Knorpelgewebe zur Identifizierung einer existierenden oder entstehenden Knorpeldegeneration herangezogen werden. Weitere geeignete Detektionsmethoden der SGK Expression sind dem zuständigen Fachmann bekannt. Geeignete Techniken zur Entnahme von Gewebeproben, z.B. Knorpel oder Synovialflüssigkeit, in der abgeschilferte Knorpelzellen enthalten sind, sowie deren Aufbereitung für die verschiedenen Analyseverfahren sind dem Fachmann ebenfalls hinlänglich bekannt.

Geeignete Antikörper zur Detektion der SGK Familie oder einzelner SGK Familienmitglieder sind kommerziell erhältlich (Santa Cruz Biotechnology, Europa Bioproducts Ltd), oder können in Kaninchen unter Verwendung von SGK Peptiden z,B. Polypeptiden mit den Sequenzen gemäß SEQ ID No. 4, 5, 6 oder SEQ ID No. 7-12 nach Standardprotokollen hergestellt werden (s. z.B. nachfolgend angegebene Standardliteratur). Ihre Herstellung wird herkömmlicherweise kommerziell von verschiedenen Anbietern vorgenommen, z.B. durch BioTrend, Köln, Deutschland. Zur Herstellung von Antiseren oder Antikörpern, die spezifisch für eines der SGK Familienmitglieder sind, werden dabei vorzugsweise Polypeptidfragmente des C-terminalen nicht katalytischen Bereiches des jeweiligen Familienmitgliedes, z.B. der 100, vorzugsweise 80 oder weniger C-terminalen Aminosäuren oder besonders bevorzugt Polypeptidfragmente ausgewählt aus den 100, vorzugsweise 85 N-terminalen Aminosäuren der SGK Proteine verwendet, insbesondere Fragmente bestehend aus 20 bis 100, 50 bis 90 und besonders bevorzugt aus den 85 N-terminalen Aminosäuren des jeweiligen SGK Familienmitglieds (bei SGK2 ausgehend von der Sequenz der SGK2β; für SGK2α eignen sich hierzu besonders die 21 N-terminalen Aminosäuren; vgl. auch Kobayashi et al., 1999b).

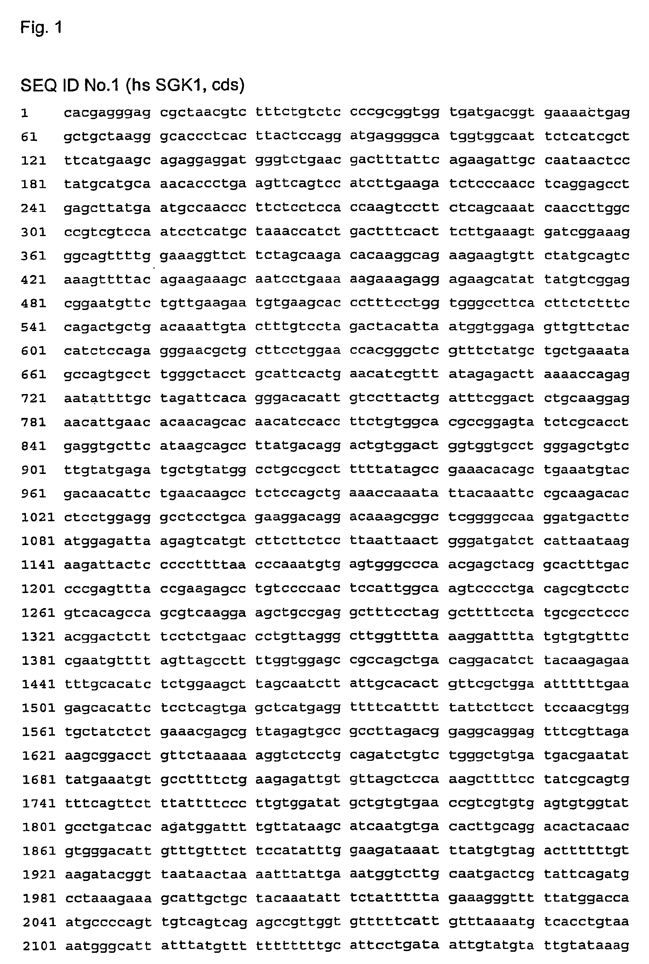

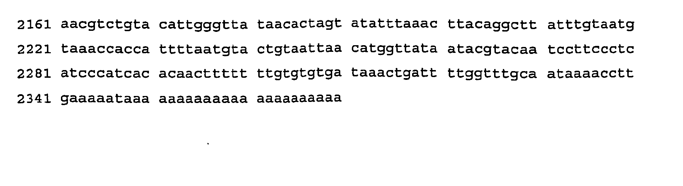

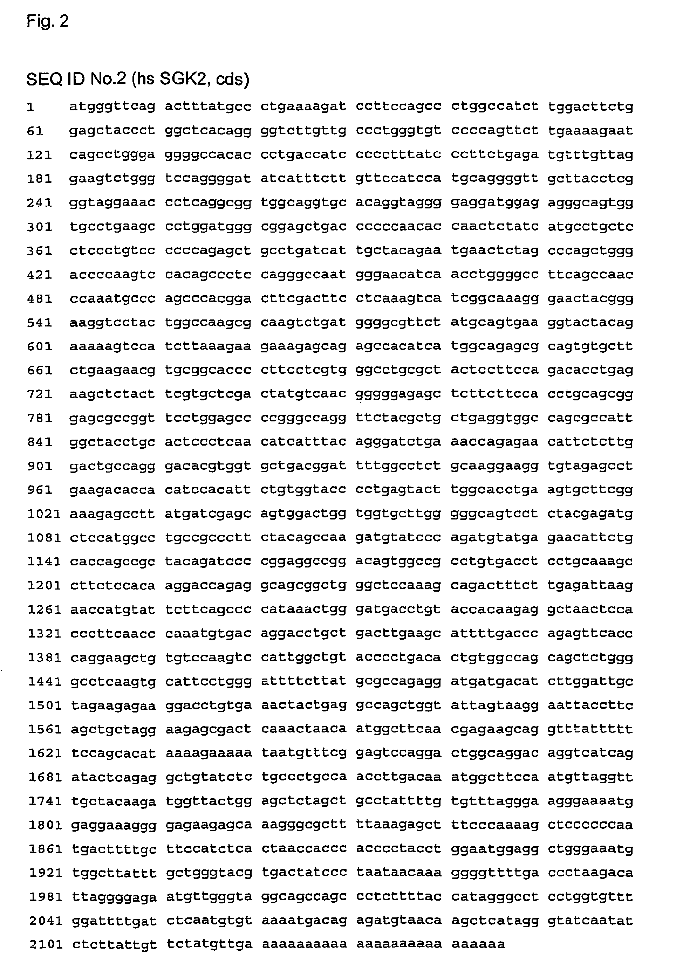

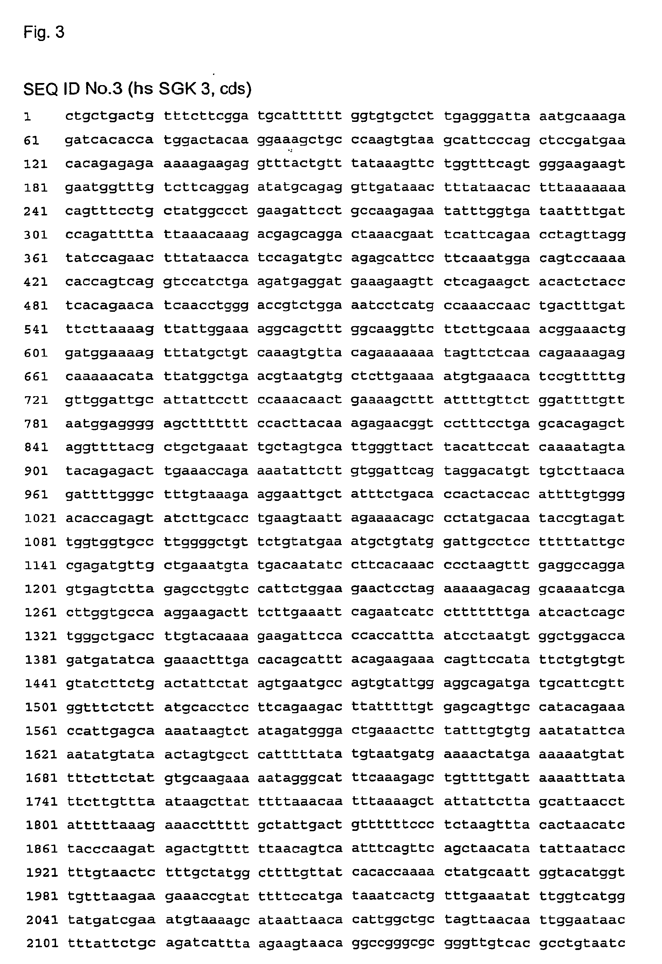

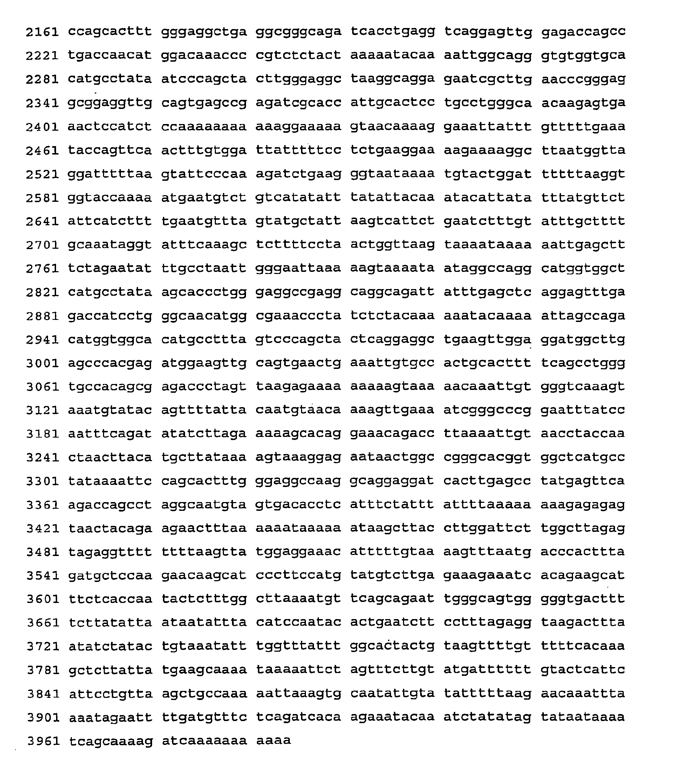

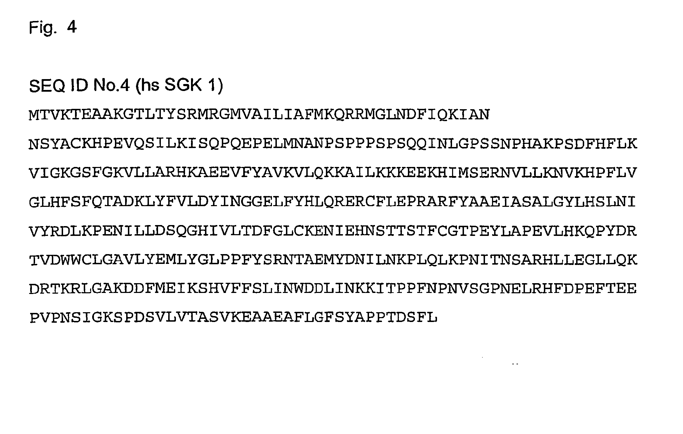

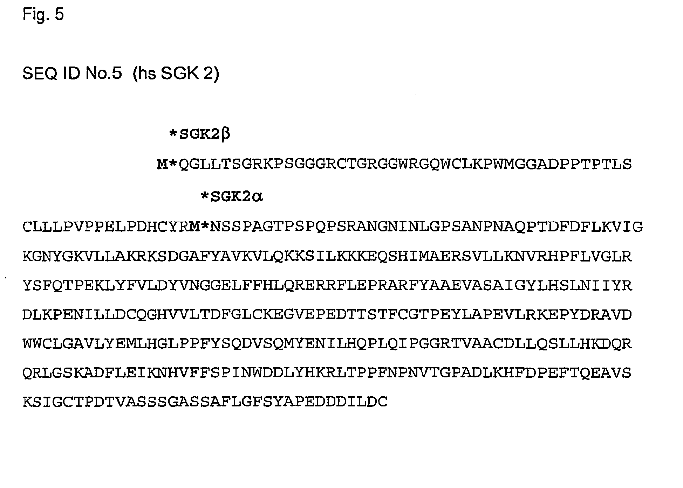

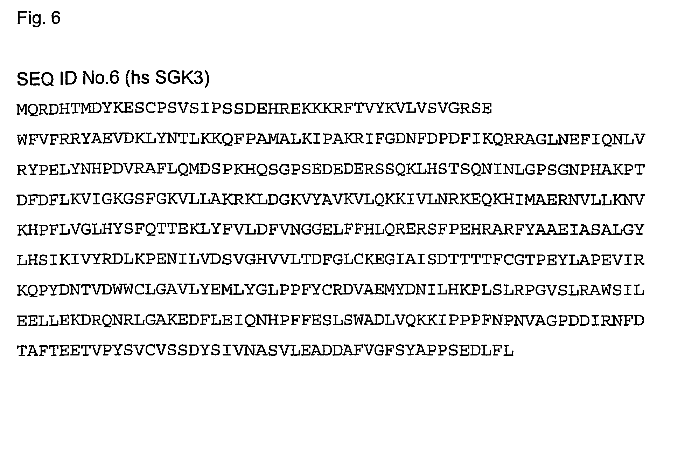

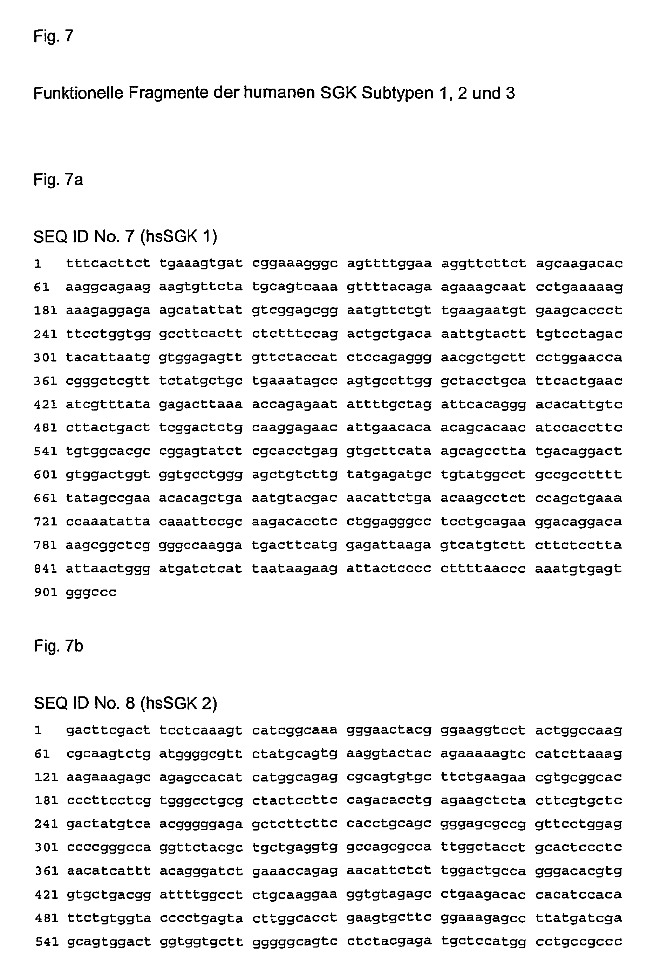

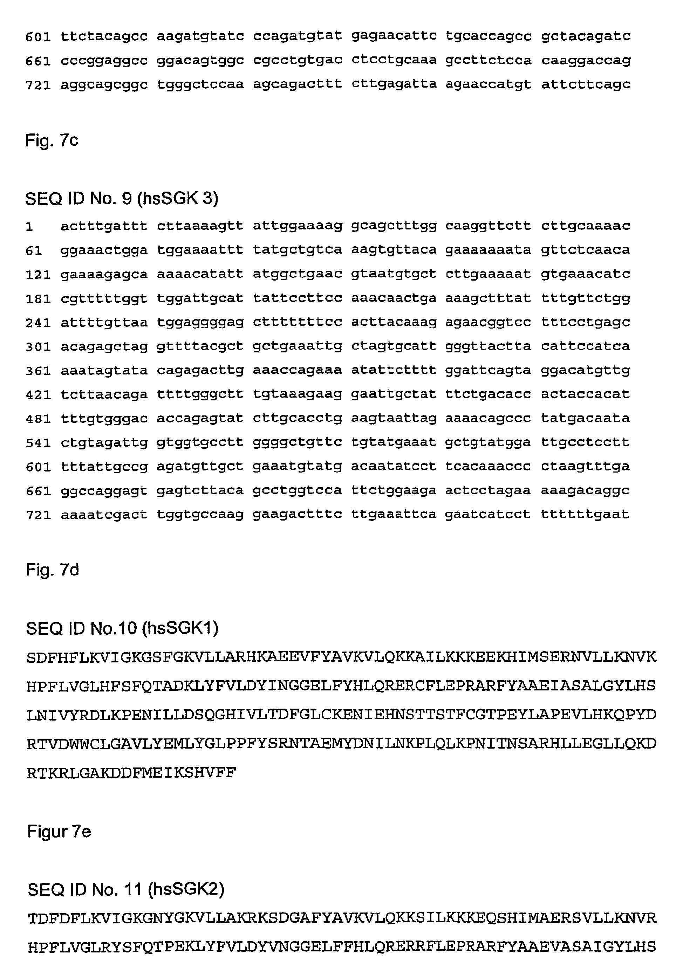

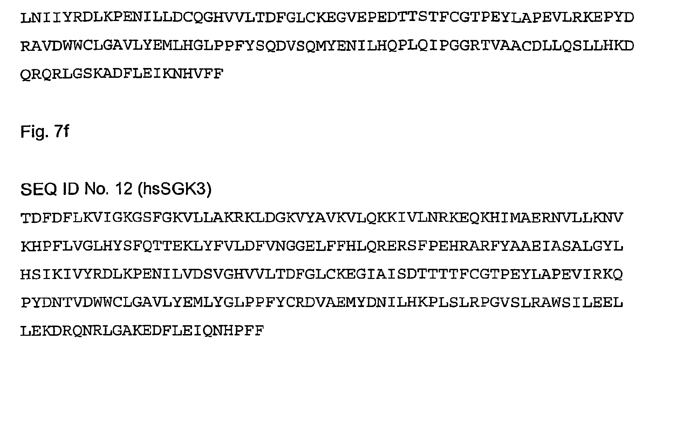

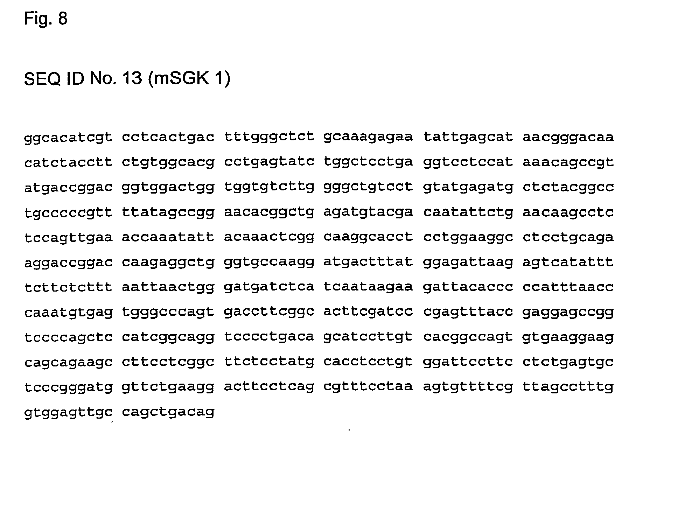

Die Auswahl und Herstellung geeigneter Sonden zur Hybridisierung immobilisierter Nukleinsäuren auf Northern und Southern Blots, DNA-Microarrays, oder Gewebeschnitten in situ sowie von Oligonukleotidprimem für die PCR ist dem zuständigen Fachmann hinlänglich bekannt (s. hierzu auch die nachfolgend angegebene Standardliteratur). Zum differentiellen Nachweis eignen sich dabei insbesondere Sonden, die für oben genannte, wenig homologe Bereiche der verschiedenen SGK Familienmitglieder kodieren, z.B. Sonden ausgewählt aus den ersten 255 Nukleotiden der kodierenden Sequenz (entsprechend den 85 wenig homologen N-terminalen Aminosäuren der verschiedenen SGK Familienmitglieder; bzw. für SGK2α Sonden ausgewählt aus den ersten 63 Nukleotiden der kodierenden Sequenz entsprechend der wenig homologen N-terminalen 21 Aminosäuren des SGK2α Proteins; s.o.) Zum Nachweis der SGK1-Expression eignen sich dabei vorzugsweise Sonden mit der Sequenz gemäß SEQ ID No. 1 (Figur 1), SEQ ID No7 (Figur 7) oder SEQ ID No. 13 (Figur 8).

Bei dem erfindungsgemäßen Verfahren wird SGK1 nachgewiesen.

Das erfindungsgemäße Verfahren zur Identifizierung degenerativer Knorpelveränderungen enthält vorzugsweise die folgenden Schritte:
a) Messung des Gehaltes an SGK Protein oder mRNA einer zu untersuchenden isolierten Knorpelprobe;
b) Vergleich mit dem Gehalt an SGK Protein oder mRNA einer isolierten Probe von nicht degeneriertem Knorpel,
wobei die Menge an SGK in degeneriertem oder degenerierendem Knorpel größer ist als die in nicht degeneriertem Knorpel.

Diese erfindungsgemäßen Verfahren erlauben die einfache und sichere Diagnose entstehender oder bestehender degenerativer Knorpelveränderungen durch entsprechende Analyse entnommener Synovialflüssigkeit oder Gewebeproben. Gemäß einer bevorzugten Ausführungsform der verschiedenen Gegenstände und Aspekte der Erfindung ist SGK ein Polynukleotid, vorzugsweise humane SGK-1 oder ein Fragment oder Derivat davon und enthält oder besteht , wie in Anspruch 12 beschrieben.

Die erfindungsgemäß verwendeten Fragmente der SGK enthalten oder bestehen vorzugsweise aus der Aminosäuresequenz gemäß SEQ ID No. 10, (Figur7).

Die Stringenz beschreibt Reaktionsbedingungen, die die Spezifität der Hybridisierung oder die Anlagerung zweier einzelsträngiger Nukleinsäuremoleküle aneinander beeinflussen. Die Stringenz und damit auch die Spezifität einer Reaktion hängt dabei unter anderem von der Temperatur und den Pufferbedingungen ab: So lässt sich die Stringenz und somit auch die Spezifität steigern, indem man die Temperatur erhöht und die Ionenstärke erniedrigt. Niedrig stringente Bedingungen (also auch geringere Reaktions- bzw. Hybridisierungsspezifität) liegen beispielsweise vor, wenn die Hybridisierung bei Raumtemperatur in 2xSSC-Lösung durchgeführt wird. Hoch stringente Bedingungen sind dagegen beispielsweise gegeben, wenn bei 68°C in 0,1X SSC und 0,1% SDS-Lösung hybridisiert wird.

Hybridisierung unter stringenten Bedingungen im Sinne dieser Anmeldung bedeutet dabei vorzugsweise
1) Hybridisierung der markierten Sonde mit der zu untersuchenden Probe bei 60 bis 70°C und vorzugsweise 65°C oder im Falle von Oligonukleotiden 5°C unter dem Schmelzpunkt des Doppelstranges aus Oligonukleotid und Probe (dieser Schmelzpunkt wird auch als "Annealingtemperatur" bezeichnet) über Nacht in 50mM Tris pH 7,5, 1 M NaCl, 1% SDS, 10% Dextran Sulfat, 0,5 mg/ml denatuerierte Herings- oder Lachssperma DNA.
2) Waschen für 10 Minuten bei Raumtemperatur in 2xSSC.
3) Waschen für 30 Minuten bei 60 bis 70°C und vorzugsweise 65°C (oder im Falle von Oligonukleotiden, 5°C unter der Annealingtemperatur) in 1x SSC / 0,1% SDS.
4) Waschen für 30 Minuten bei bei 60 bis 70°C und vorzugsweise 65°C (oder im Falle von Oligonukleotiden, 5°C unter der Annealingtemperatur) in 0,2 x SSC / 0,1 % SDS.
5) Waschen für 30 Minuten bei bei 60 bis 70°C und vorzugsweise 65°C (oder im Falle von Oligonukleotiden, 5°C unter der Annealingtemperatur) in 0,1 x SSC / 0,1 % SDS.

Oligonukleotide sind vorzugsweise DNA-Fragmente mit einer Länge zwischen 15 bis 30, vorzugsweise 20 Nukleotiden. Die Annealingtemperatur wird dabei durch die Formel Tm=2 x (Anzahl A+T) + 4 x (Anzahl G+C)°C bestimmt. Zur Herstellung einer 2 x SSC bzw. 0,1 x SSC-Lösung wird beispielsweise eine 20 x SSC-Lösung entsprechend verdünnt. Die 20 x SSC-Lösung besteht aus: 3M NaCl/0,3 M Na-Citrat x 2H₂O.

Vor der Durchführung der Hybridisierung werden die zu untersuchenden Polynukleotide, ggf. nach elektrophoretischer Auftrennung (sog. Southern (DNA) oder Northern Blot (RNA)), oder ohne elektrophoretische Auftrennung (sog. Dot oder Slot Blot) auf eine geeignete Membran, z.B. eine Nylon- oder Nitrozellulosemembran übertragen. Die Hybridisierung erfolgt mit einer auf geeignete Weise markierten Sonde. So sind die radioaktive Markierung oder Markierung mit Fluoreszenzfarbstoffen zweckmässig, andere Markierungsarten sind ebenfalls denkbar. Die Sonde ist ein einzelsträngiges Polyribo- oder Polydesoxyribonukleotid, welches per se einzelsträngig vorliegt oder gewöhnlicherweise doppelsträngig ist, aber in denaturiertem Zustand eingesetzt wird. Diese Sonde bindet durch Basenpaarung an die ihrerseits einzelsträngig vorliegende DNA- oder RNA- Probe.

Die verschiedenen Aminosäure- und Nukleotidsequenzen sind den Figuren zu entnehmen.

Die verschiedenen Gegenstände und Aspekte der Erfindung eignen sich besonders zum Einsatz bei arthritischen Erkrankungen, vorzugsweise der Osteoarthrose oder Rheumatoiden Arthritis und insbesondere der Osteoarthrose.

### Literatur

Akutsu,N., Lin,R., Bastien,Y., Bestawros,A., Enepekides,D.J., Black,M.J., and White,J.H. (2001). Regulation of gene Expression by 1alpha,25-dihydroxyvitamin D3 and Its analog EB1089 under growth-inhibitory conditions in squamous carcinoma Cells. Mol Endocrinol., 15, 1127-1139.
Ali,S., Wei,Y., Lerea,K.M., Becker,L., Rubin,C.S., and Wang,W. (2001). PKA-induced stimulation of ROMK1 channel activity is governed by both tethering and nontethering domains of an A kinase anchor protein. Cell Physiol Biochem., 11, 135-142.
Alliston,T.N., Gonzalez-Robayna,I.J., Buse,P., Firestone,G.L., and Richards,J.S. (2000). Expression and localization of serum/glucocorticoid-induced kinase in the rat ovary: relation to follicular growth and differentiation. Endocrinology, 141, 385-395.
Alliston,T.N., Maiyar,A.C., Buse,P., Firestone,G.L., and Richards,J.S. (1997). Follicle stimulating hormone-regulated expression of serum/glucocorticoid-inducible kinase in rat ovarian granulosa cells: a functional role for the Sp1 family in promoter activity. Mol Endocrinol., 11, 1934-1949.
Alvarez,d.l.R., Zhang,P., Naray-Fejes-Toth,A., Fejes-Toth,G., and Canessa,C.M. (1999). The serum and glucocorticoid kinase sgk increases the abundance of epithelial sodium channels in the plasma membrane of Xenopus oocytes. J Biol Chem, 274, 37834-37839.
Bhargava,A., Fullerton,M.J., Myles,K., Purdy,T.M., Funder,J.W., Pearce,D., and Cole,T.J. (2001). The serum- and glucocorticoid-induced kinase is a physiological mediator of aldosterone action. Endocrinology, 142, 1587-1594.
Biondi,R.M., Kieloch,A., Currie,R.A., Deak,M., and Alessi,D.R. (2001). The PIF-binding pocket in PDK1 is essential for activation of S6K and SGK, but not PKB. EMBO J, 20, 4380-4390.
Boehmer,C., Okur,F., Setiawan,I., Broer,S., and Lang,F. (2003a). Properties and regulation of glutamine transporter SN1 by protein kinases SGK and PKB. Biochem. Biophys. Res. Commun., 306, 156-162.
Boehmer,C., Wilhelm,V., Palmada,M., Wallisch,S., Henke,G., Brinkmeier,H., Cohen,P., Pieske,B., and Lang,F. (2003b). Serum and glucocorticoid inducible kinases in the regulation of the cardiac sodium channel SCN5A. Cardiovasc Res., 57, 1079-1084.
Boehmer,C., Wagner,C.A., Beck,S., Moschen,I., Melzig,J., Werner,A., Lin,J.T., Lang,F., and Wehner,F. (2000). The shrinkage-activated Na(+) conductance of rat hepatocytes and its possible correlation to rENaC. Cell Physiol Biochem., 10, 187-194.
Brennan,F.E. and Fuller,P.J. (2000). Rapid upregulation of serum and glucocorticoid-regulated kinase (sgk) gene expression by corticosteroids in vivo. Mol Cell Endocrinol., 166, 129-136.
Chen,S.Y., Bhargava,A., Mastroberardino,L., Meijer,O.C., Wang,J., Buse,P., Firestone,G.L., Verrey,F., and Pearce,D. (1999). Epithelial sodium channel regulated by aldosterone-induced protein sgk. Proc. Natl. Acad. Sci U. S. A, 96, 2514-2519.
Cooper,M.S., Bujalska,I., Rabbitt,E., Walker,E.A., Bland,R., Sheppard,M.C., Hewison,M., and Stewart,P.M. (2001). Modulation of 11 beta-hydroxysteroid dehydrogenase isozymes by proinflammatory cytokines in osteoblasts: an autocrine switch from glucocorticoid inactivation to activation. J Bone Miner. Res., 16, 1037-1044.
Davies,S.P., Reddy,H., Caivano,M., and Cohen,P. (2000). Specificity and mechanism of action of some commonly used protein kinase inhibitors. Biochem. J, 351, 95-105.
Debonneville,C., Flores,S.Y., Kamynina,E., Plant,P.J., Tauxe,C., Thomas,M.A., Munster,C., Chraibi,A., Pratt,J.H., Horisberger,J.D., Pearce,D., Loffing,J., and Staub,O. (2001). Phosphorylation of Nedd4-2 by Sgk1 regulates epithelial Na(+) channel cell surface expression. EMBO J, 20, 7052-7059.
Delmolino,L.M. and Castellot,J.J., Jr. (1997). Heparin suppresses sgk, an early response gene in proliferating vascular smooth muscle cells. J Cell Physiol, 173, 371-379.
Djelidi,S., Beggah,A., Courtois-Coutry,N., Fay,M., Cluzeaud,F., Viengchareun,S., Bonvalet,J.P., Farman,N., and Blot-Chabaud,M. (2001). Basolateral translocation by vasopressin of the aldosterone-induced pool of latent Na-K-ATPases is accompanied by alpha1 subunit dephosphorylation: study in a new aldosterone-sensitive rat cortical collecting duct cell line. J Am. Soc. Nephrol., 12, 1805-1818.
Eisen, M.B. Spellman, P.T., Brown. P.O., Botstein, D. (1998). Cluster analysis and display of genome-wide expression patterns. Proc. Natl. Acad. Sci. USA 95(25):14863-14868.
Embark,H.M., Bohmer,C., Vallon,V., Luft,F., and Lang,F. (2003). Regulation of KCNE1-dependent K(+) current by the serum and glucocorticoid-inducible kinase (SGK) isoforms. Pflugers Arch, 445, 601-606.
Faletti,C.J., Perrotti,N., Taylor,S.I., and Blazer-Yost,B.L. (2002). sgk: an essential convergence point for peptide and steroid hormone regulation of ENaC-mediated Na+ transport. Am. J Physiol Cell Physiol, 282, C494-C500.
Fillon, S., Warntges, S., Matskevitch, J., Moschen, I., Setiawan, I., Gamper, N., Feng, Y.X., Stegen, C., Friedrich, B., Waldegger, S., Broer, S., Wagner, C.A., Huber, S.M., Klingel, K., Vereninov, A., Lang, F. (2001). Serum- and glucocorticoid-dependent kinase, cell volume, and the regulation of epithelial transport. Comp. Biochem. Physiol. A Mol. Integr. Physiol. 130(3):367-376.
Fillon,S., Klingel,K., Warntges,S., Sauter,M., Gabrysch,S., Pestel,S., Tanneur,V., Waldegger,S., Zipfel,A., Viebahn,R., Haussinger,D., Broer,S., Kandolf,R., and Lang,F. (2002). Expression of the serine/threonine kinase hSGK1 in chronic viral hepatitis. Cell Physiol Biochem., 12, 47-54. Ref ID: 1
Firestone,G.L., Giampaolo,J.R., and O'Keeffe,B.A. (2003). Stimulus-dependent regulation of serum and glucocorticoid inducible protein kinase (SGK) transcription, subcellular localization and enzymatic activity. Cell Physiol Biochem., 13, 1-12.
Friedrich, B., Feng, Y., Cohen, P., Risler, T., Vandewalle, A., Broer, S., Wang, J., Pearce, D., Lang, F. (2003). The serine/threonine kinases SGK2 and SGK3 are potent stimulators of the epithelial Na(+) channel alpha, beta, gamma-EnaC. Pflugers Arch. 445(6):693-696.
Funder,J. (2001). Mineralocorticoids and cardiac fibrosis: the decade in review. Clin Exp Pharmacol Physiol, 28, 1002-1006.
Gamper,N., Fillon,S., Feng,Y., Friedrich,B., Lang,P.A., Henke,G., Huber,S.M., Kobayashi,T., Cohen,P., and Lang,F. (2002a). K+ channel activation by all three isoforms of serum- and glucocorticoid-dependent protein kinase SGK. Pflugers Arch, 445, 60-66.
Gamper,N., Fillon,S., Huber,S.M., Feng,Y., Kobayashi,T., Cohen,P., and Lang,F. (2002b). IGF-1 up-regulates K+ channels via PI3-kinase, PDK1 and SGK1. Pflugers Arch, 443, 625-634.
Giebisch,G. (1998). Renal potassium transport: mechanisms and regulation. Am. J Physiol, 274, F817-F833.
Gonzalez-Robayna,I.J., Alliston,T.N., Buse,P., Firestone,G.L., and Richards,J.S. (1999). Functional and subcellular changes in the A-kinase-signaling pathway: relation to aromatase and Sgk expression during the transition of granulosa cells to luteal cells. Mol Endocrinol., 13, 1318-1337.
Gonzalez-Robayna,I.J., Falender,A.E., Ochsner,S., Firestone,G.L., and Richards,J.S. (2000). Follicle-Stimulating hormone (FSH) stimulates phosphorylation and activation of protein kinase B (PKB/Akt) and serum and glucocorticoid-Induced kinase (Sgk): evidence for A kinase-independent signaling by FSH in granulosa cells. Mol Endocrinol., 14, 1283-1300.
Hayashi, M., Tapping, R.I., Chao, T.H., Lo, J.F., King, C.C., Yang, Y., Lee, J.D. (2001). BMK1 mediates growth factor-induced cell proliferation through direct cellular activation of serum and glucocorticoid-inducible kinase. J. Biol. Chem. 276(12):8631-8634.
Imaizumi,K., Tsuda,M., Wanaka,A., Tohyama,M., and Takagi,T. (1994). Differential expression of sgk mRNA, a member of the Ser/Thr protein kinase gene family, in rat brain after CNS injury. Brain Res Mol Brain Res, 26, 189-196.
Klingel,K., Warntges,S., Bock,J., Wagner,C.A., Sauter,M., Waldegger,S., Kandolf,R., and Lang,F. (2000). Expression of cell volume-regulated kinase h-sgk in pancreatic tissue. Am. J Physiol Gastrointest. Liver Physiol, 279, G998-G1002.
Kobayashi,T. and Cohen,P. (1999a). Activation of serum- and glucocorticoid-regulated protein kinase by agonists that activate phosphatidylinositide 3-kinase is mediated by 3-phosphoinositide-dependent protein kinase-1 (PDK1) and PDK2. Biochem. J, 339 (Pt 2), 319-328.
Kobayashi,T., Deak,M., Morrice,N., and Cohen,P. (1999b). Characterization of the structure and regulation of two novel isoforms of serum- and glucocorticoid-induced protein kinase. Biochem. J, 344 Pt 1, 189-197.
Kumar,J.M., Brooks,D.P., Olson,B.A., and Laping,N.J. (1999). Sgk, a putative serine/threonine kinase, is differentially expressed in the kidney of diabetic mice and humans. J Am. Soc. Nephrol., 10, 2488-2494.
Lang, F., Cohen, P. (2001). The regulation and physiological roles of serum and glucocorticoid-induced protein kinase. Sci. STKE (108):RE17
Lang,F., Klingel,K., Wagner,C.A., Stegen,C., Warntges,S., Friedrich,B., Lanzendorfer,M., Melzig,J., Moschen,I., Steuer,S., Waldegger,S., Sauter,M., Paulmichl,M., Gerke,V., Risler,T., Gamba,G., Capasso,G., Kandolf,R., Hebert,S.C., Massry,S.G., and Broer,S. (2000). Deranged transcriptional regulation of cell-volume-sensitive kinase hSGK in diabetic nephropathy. Proc. Natl. Acad. Sci U. S. A, 97, 8157-8162.
Lee, E., Lein, E.S., Firestone, G.L. (2001). Tissue-specific expression of the transcriptionally regulated serum and glucocorticoid-inducible protein kinase (sgk) during einbryogenesis. Mech. Dev. 103(1-2):177-81
Liu,D., Yang,X., and Songyang,Z. (2000). Identification of CISK, a new member of the SGK kinase family that promotes IL-3-dependent survival. Curr. Biol, 10, 1233-1236.
Loffing,J., Zecevic,M., Feraille,E., Kaissling,B., Asher,C., Rossier,B.C., Firestone,G.L., Pearce,D., and Verrey,F. (2001). Aldosterone induces rapid apical translocation of ENaC in early portion of renal collecting system: possible role of SGK. Am. J Physiol Renal Physiol, 280, F675-F682
Maier et al., 1997; in: Automation for genome characterisation. Ed T. J. Beueisdijk.; Publishers: J. Wiley, New York
Meier-Ewert, S. et al., Sebastian. Maier Elmar. Ahmadi Ali. Curtis Jon. Lehrach Hans. An automated approach to generating expressed sequence catalogues. Nature (London). 361 (6410). 1993. 375-376
Mikosz,C.A., Brickley,D.R., Sharkey,M.S., Moran,T.W., and Conzen,S.D. (2001). Glucocorticoid receptor-mediated protection from apoptosis is associated with induction of the serine/threonine survival kinase gene, sgk-1. J Biol Chem, 276, 16649-16654.
Mizuno,H. and Nishida,E. (2001). The ERK MAP kinase pathway mediates induction of SGK (serum- and glucocorticoid-inducible kinase) by growth factors. Genes Cells, 6, 261-268.
Naray-Fejes-Toth,A., Canessa,C., Cleaveland,E.S., Aldrich,G., and Fejes-Toth,G. (1999). sgk is an aldosterone-induced kinase in the renal collecting duct. Effects on epithelial na+ channels. J Biol Chem, 274, 16973-16978.
Naray-Fejes-Toth,A., Fejes-Toth,G., Volk,K.A., and Stokes,J.B. (2000). SGK is a primary glucocorticoid-induced gene in the human. J Steroid Biochem. Mol Biol, 75, 51-56**.**
Park,J., Leong,M.L., Buse,P., Maiyar,A.C., Firestone,G.L., and Hemmings,B.A. (1999). Serum and glucocorticoid-inducible kinase (SGK) is a target of the PI 3-kinasestimulated signaling pathway. EMBO J, 18, 3024-3033.
Perrotti,N., He,R.A., Phillips,S.A., Haft,C.R., and Taylor,S.I. (2001). Activation of serum- and glucocorticoid-induced protein kinase (Sgk) by cyclic AMP and insulin. J Biol Chem, 276, 9406-9412.
Richards,J.S., Fitzpatrick,S.L., Clemens,J.W., Morris,J.K., Alliston,T., and Sirois,J. (1995). Ovarian cell differentiation: a cascade of multiple hormones, cellular signals, and regulated genes. Recent Prog. Horm. Res, 50, 223-254.
Rozansky,D.J., Wang,J., Doan,N., Purdy,T., Faulk,T., Bhargava,A., Dawson,K., and Pearce,D. (2002). Hypotonic induction of SGK1 and Na+ transport in A6 cells. Am. J Physiol Renal Physiol, 283, F105-F113.
Shenolikar,S. and Weinman,E.J. (2001). NHERF: targeting and trafficking membrane proteins. Am. J Physiol Renal Physiol, 280, F389-F395. Ref ID: 16
Shigaev,A., Asher,C., Latter,H., Garty,H., and Reuveny,E. (2000). Regulation of sgk by aldosterone and its effects on the epithelial Na(+) channel. Am. J Physiol Renal Physiol, 278, F613-F619.
Shukunami,C., Shigeno,C., Atsumi,T., Ishizeki,K., Suzuki,F., and Hiraki,Y. (1996). Chondrogenic differentiation of clonal mouse embryonic cell line ATDC5 in vitro: differentiation-dependent gene expression of parathyroid hormone (PTH)/PTHrelated peptide receptor. J Cell Biol, 133, 457-468.
Shukunami,C., Ishizeki,K., Atsumi,T., Ohta,Y., Suzuki,F., and Hiraki,Y. (1997). Cellular hypertrophy and calcification of embryonal carcinoma-derived chondrogenic cell line ATDC5 in vitro. J Bone Miner. Res, 12, 1174-1188.
Snyder,P.M., Olson,D.R., and Thomas,B.C. (2002). Serum and glucocorticoid-regulated kinase modulates Nedd4-2-mediated inhibition of the epithelial Na+ channel. J Biol Chem, 277, 5-8.
Staub,O., Gautschi,I., Ishikawa,T., Breitschopf,K., Ciechanover,A., Schild,L., and Rotin,D. (1997). Regulation of stability and function of the epithelial Na+ channel (ENaC) by ubiquitination. EMBO J, 16, 6325-6336.
Vincent,G.M. (1998). The molecular genetics of the long QT syndrome: genes causing fainting and sudden death. Annu. Rev. Med, 49, 263-274**.**
Wagner,C.A., Broer,A., Albers,A., Gamper,N., Lang,F., and Broer,S. (2000). The heterodimeric amino acid transporter 4F2hc/LAT1 is associated in Xenopus oocytes with a non-selective cation channel that is regulated by the serine/threonine kinase sgk-1. J Physiol, 526 Pt 1, 35-46.
Wagner,C.A., Ott,M., Klingel,K., Beck,S., Melzig,J., Friedrich,B., Wild,K.N., Broer,S., Moschen,I., Albers,A., Waldegger,S., Tummler,B., Egan,M.E., Geibel,J.P., Kandolf,R., and Lang,F. (2001). Effects of the serine/threonine kinase SGK1 on the epithelial Na(+) channel (ENaC) and CFTR: implications for cystic fibrosis. Cell Physiol Biochem., 11, 209-218.
Wald,H., Garty,H., Palmer,L.G., and Popovtzer,M.M. (1998). Differential regulation of ROMK expression in kidney cortex and medulla by aldosterone and potassium. Am. J Physiol, 275, F239-F245.
Waldegger,S., Barth,P., Raber,G., and Lang,F. (1997). Cloning and characterization of a putative human serine/threonine protein kinase transcriptionally modified during anisotonic and isotonic alterations of cell volume. Proc. Natl. Acad. Sci U. S. A, 94, 4440-4445.
Waldegger,S., Gabrysch,S., Barth,P., Fillon,S., and Lang,F. (2000). h-sgk serine-threonine protein kinase as transcriptional target of p38/MAP kinase pathway in HepG2 human hepatoma cells. Cell Physiol Biochem., 10, 203-208.
Waldegger,S., Klingel,K., Barth,P., Sauter,M., Rfer,M.L., Kandolf,R., and Lang,F. (1999). h-sgk serine-threonine protein kinase gene as transcriptional target of transforming growth factor beta in human intestine. Gastroenterology, 116, 1081-1088.
Wang,J., Barbry,P., Maiyar,A.C., Rozansky,D.J., Bhargava,A., Leong,M., Firestone,G.L., and Pearce,D. (2001). SGK integrates insulin and mineralocorticoid regulation of epithelial sodium transport. Am. J Physiol Renal Physiol, 280, F303-F313.
Wang,W. (1999). Regulation of the ROMK channel: interaction of the ROMK with associate proteins. Am. J Physiol, 277, F826-F831.
Warntges,S., Friedrich,B., Henke,G., Duranton,C., Lang,P.A., Waldegger,S., Meyermann,R., Kuhl,D., Speckmann,E.J., Obermuller,N., Witzgall,R., Mack,A.F., Wagner,H.J., Wagner,A., Broer,S., and Lang,F. (2002a). Cerebral localization and regulation of the cell volume-sensitive serum- and glucocorticoid-dependent kinase SGK1. Pflugers Arch, 443, 617-624.
Warntges,S., Klingel,K., Weigert,C., Fillon,S., Buck,M., Schleicher,E., Rodemann,H.P., Knabbe,C., Kandolf,R., and Lang,F. (2002b). Excessive transcription of the human serum and glucocorticoid dependent kinase hSGK1 in lung fibrosis. Cell Physiol Biochem., 12, 135-142.
Webster,M.K., Goya,L., and Firestone,G.L. (1993a). Immediate-early transcriptional regulation and rapid mRNA turnover of a putative serine/threonine protein kinase. J Biol Chem, 268, 11482-11485.
Webster,M.K., Goya,L., Ge,Y., Maiyar,A.C., and Firestone,G.L. (1993b). Characterization of sgk, a novel member of the serine/threonine protein kinase gene family which is transcriptionally induced by glucocorticoids and serum. Mol Cell Biol, 13, 2031-2040.
Wulff,P., Vallon,V., Huang,D.Y., Volkl,H., Yu,F., Richter,K., Jansen,M., Schlunz,M., Klingel,K., Loffing,J., Kauselmann,G., Bosl,M.R., Lang,F., and Kuhl,D. (2002). Impaired renal Na(+) retention in the sgk1-knockout mouse. J Clin Invest, 110, 1263-1268.
Yun,C.C. (2003). Concerted roles of SGK1 and the Na+/H+ exchanger regulatory factor 2 (NHERF2) in regulation of NHE3. Cell Physiol Biochem., 13, 29-40.
Yun,C.C., Chen,Y., and Lang,F. (2002). Glucocorticoid activation of Na(+)/H(+) exchanger isoform 3 revisited. The roles of SGK1 and NHERF2. J Biol Chem, 277, 7676-7683.

### Literatur zu Standardmethoden:

Falls nicht anders angegeben, sind oder können die vorliegend beschriebenen Labormethoden gemäß der nachfolgend aufgeführten Standardliteratur durchgeführt werden.
Bancroft et al., 1999; Methods in Microbiology 28: 67-82.
Sambrook et al. (1989) Molecular Cloning: A Laboratory Manual. Second edition. Cold Spring Harbor Laboratory Press, Cold Spring Harbor, NY. 545 pp;
Current Protocols in Molecular Biology; regularly updated, e.g. Volume 2000; Wiley & Sons, Inc; Editors: Fred M. Ausubel, Roger Brent, Robert Eg. Kingston, David D. Moore, J.G. Seidman, John A. Smith, Kevin Struhl.
Current Protocols in Human Genetics; regularly uptdated; Wiley & Sons, Inc; Editors: Nicholas C. Dracopoli, Honathan L. Haines, Bruce R. Korf, Cynthia C. Morton, Christine E. Seidman, J.G. Seigman, Douglas R. Smith.
Current Protocols in Protein Science; regularly updated; Wiley & Sons, Inc; Editors: John E. Coligan, Ben M. Dunn, Hidde L. Ploegh, David W. Speicher, Paul T. Wingfield. Molecular Biology of the Cell; third edition; Alberts, B., Bray, D., Lewis, J., Raff, M., Roberts, K., Watson, J.D.; Garland Publishing, Inc. New York & London, 1994;
Short Protocols in Molecular Biology, 5th edition, by Frederick M. Ansubel (Editor), Roger Brent (Editor), Robert E. Kingston (Editor), David D. Moore (Editor), J.G. Seidman (Editor), John A. Smith (Editor), Kevin Struhl (Editor), October 2002, John Wiley & Sons, Inc., New York"
Gene Targeting: apractical approach (1995), Editor: A.L. Joyner, IRL Press. Transgenic Animal Technology A Laboratory Handbook. C.A. Pinkert, editor; Academic Press Inc., San Diego, California, 1994 (ISBN: 0125571658).

Die Erfindung wird nachfolgend anhand von Beispielen und Figuren, die die Gegenstände der vorliegenden Erfindung jedoch nicht einschränken sollen, ausführlich erläutert:

### Beispiel 1:

### Genexpressionsanalysen mittels Osteoarthrose-Arrays

Grundlage der Genexpressionsanalysen war ein von der Firma GPC-Biotech auftragsgemäss hergestellter osteoarthrose-spezifischer cDNA-Array, der aus 4500 Knorpel-relevanten Genen besteht, die auf Nylonmembranen aufgetragen wurden. Als Sonde zur Hybridisierung des Arrays wurde gesamt-RNA (Herstellung nach Standardmethoden) aus gesundem und osteoarthrotischem Gelenkknorpel durch reverse Transkription unter Inkorporation ³²P markierter Nukleotide in eine radioaktive cDNA übersetzt und mit den cDNA-Proben des Arrays nach Standardmethoden inkubiertNach der Hybridisierung und anschließenden Waschen der Membranen wurden die Hybridisierungssignale durch Autoradiographie detektiert. Von Genen, die differentiell unterschiedlich zwischen gesundem und osteoarthrotischem Knorpel exprimiert waren, wurden 270 für weiterführende Analysen in Betracht gezogen. Eines dieser Gene war überraschenderweise SGK:

Die Befunde der Genexpressionsanalyse der Erfinder zeigten dabei erstmals, dass es bei der Osteoarthrose im Gelenkknorpel zu einer Expressionsinduktion von SGK kommt. Dieser Befund konnte weiter durch die Anzahl von Klonen in cDNA-Banken konfirmiert werden: Dabei zeigte sich, dass im Gegensatz zu Bibliotheken von normalem Knorpel, SGK-cDNA in cDNA Bibliotheken osteoarthrotischen Knorpels 14 mal so häufig exprimiert ist.

### Beispiel 2:

### Herstellung der Arrays und Hybridisierung

Die Klone zur Herstellung der Arrays wurden durch SSH (Subtractive Suppression Hybridization) ausgewählt. Desweiteren wurden Kontroll Klone und Klone von Housekeeping Genen verwendet. Die Amplifikation erfolgte unter Verwendung eines PCR Kits der Firma Qiagen, Hilden UK in 384-well Polypropylen Platten (Genetix, UK) unter Verwendung des M13 forward (5' GCT ATT ACG CCA GCT GGC GAA AGG GGG ATG TG 3') und M13 reverse Primers (5' CCC CAG GCT TTA CAC TTT ATG CTT CCG GCT CG 3') in einer PCR Reaktion mit 50 µl Reaktionsvolumen nach Angaben des Herstellers (Qiagen, Hilden UK, s.o). Die Reaktionsansätze wurden unter Verwendung eines 384-Pin Transfergerätes (Genetix) mit 2 µl oder weniger Bakterienkultur inokuliert und die Platten anschliessend mit Plastikfolie (Genetix) versiegelt. Die PCR Amplifikation wurde in einem automatisierten Wasserbad System (KBioSystems, Basildon UK) unter Standard Bedingungen durchgeführt (Meier-Ewert, S. et al.). Die PCR Produkte von 4396 cDNA Klonen wurden einzeln auf 8 x 12 cm Replika Nylon Membranen (Hybond N+, Amersham Pharmacia Biotech) unter Verwendung eines Auftragungsroboters nach Maier et al., transferiert und anschliessend durch Lufttrocknung auf der Membran immobilisiert. Die PCR Produkte wurden dabei zweifach in Form eines 5 x 5 Musters mit einem Punkt - zu - Punkt Abstand von 850 µm auf die Membranen aufgebracht.

### Beispiel 3:

### Sondenmarkierung, Hybridisierung und Waschen

Eine Gesamtmenge von 2 µg gesamt RNA wurden unter Verwendung von Superscript II Reverser Transkriptase (#18064-071, Invitrogen) in vier verschiedenen Reaktionsansätzen in cDNA umgeschrieben. Die Ansätze enthielten dabei jeweils: 200 Einheiten Superscript II (#18064-071, Invitrogen), 50 µCi 33P α-dCTP (#NEG313H, NEN Life Science Products GmbH) jeweils 0.66 mM dATP, dGTP, dTTP, 0.5 µg Zufallshexamere (Amersham Pharmacia Biotech) und 10 mM DTT in Superscript II Erststrangreaktionspuffer (Invitrogen). Die Reaktion wurde für 4 Stunden bei 42°C inkubiert; anschliessend erfolgte die cDNA Aufreinigung unter verwendung von Quick Spin Columns (Roche, Penzberg Germany) nach Angaben des Herstellers. Vor der Hybridisierung wurde jede Sonde unter Verwendung von 0.11 Volumina Denaturierungslösung (1 M NaOH / 10 mM EDTA) und 5 µg humaner COT-1 DNA (15279-011, Invitrogen), bei 70°C für 20 Minuten denaturiert. Anschliessend wurde jede Sonde durch Zufügung einer Volumenmenge an Neutralisierungslösung (1 M Naphosphate / pH7.0), bei 70°C für 10 Minuten neutralisiert und anschliessend dem Hybridisierungsansatz zugefügt. Als Sonde wurde-revers transkribierte cDNA von Knorpel Gesamt-RNA eingesetzt.

Für jedes Hybridisierungsexperiment wurden 3 Arrays für 2 Stunden in 50 ml Hybridisierungspuffer (7 % SDS, 50 % deionisiertes Formamid, 5 x SSC, 50 mM Na-Phosphat Puffer pH 7.0 und 2 % Trockenmilchpulver) vor-hybridisiert, bevor die Sonde zugegeben wurde. Die Hybridisierung erfolgte nach Zugabe der Sonde in Plastikgefäßen (20 cm x 10 cm) bei 50°C für 16 Stunden. Nach der Hybridisierung, wurden die Arrays zwei Mal in einem Puffer aus 2 x SSC, 1 % SDS und zwei Mal in einem Puffer aus 0.1 % SSC und 0.5 % SDS gewaschen. Jeder Wasschschritt wurde 65°C für 15 Minuten durchgeführt. Anschliessend wurden die Membranen in Frischhaltefolie gepackt, 5 Tage in Kontakt mit Phospho-Storage Screens gebracht und unter Verwendung eines FUJI BAS 5000 Bilderzeugungssystems bei einer Auflösung von 25 µm gescannt. Die Bildanalyse erfolgte unter Verwendung eines handelsüblichen Array Analyse Programms (VisualGrid, GPC Biotech AG), welches die durchschnittliche Signalintensität jeder auf den Array aufgetragenen Position in computerlesbare Daten umwandelte und als Datei auf einen Datenträger speicherte.

### Beispiel 4:

### Datenanalyse

Die Rohdaten der Dateien, welche die Signalintensität der verschiedenen Array Signale (die jeweils einzelnen Klonen entsprachen) wurden folgendermassen analysiert: Zunächst wurde das Hintergrundsignal durch Abzug von Signalintensitätswerten, die von nicht mit DNA beladenen Positionen stammten, korrigiert. Anschliessend wurden unter Berücksichtigung der sechs verschiedenen Replika-Werte pro Klon (3 Arrays mit jeweils zwei Datenpunkten) korrigierte, logarithmisch transformierte Durchschnittssignalintensitäten und Standard Abweichungen für jeden der Klone auf dem Array bestimmt. Dann wurden mögliche Proben outlieers? durch Zusammenfassung von Daten einzelner Patientenproben unter Verwendung eines hierarchischen Cluster-Erstellungs Algorithmus, der auf der average-linkage Methode (Eisen et al, 1998) basiert, identifiziert. Schliesslich wurden differentiell exprimierte Klone durch Vergleich der erhaltenen Durchschnittssignalintensitäten (Vergleich der mittleren Signalintensität eines bestimmten Klones in einer cDNA Probe aus Knorpel von OA Patienten mit der mittleren Signalintensität in einer cDNA Probe aus gesundem Knorpel) identifiziert. Alternativ wurde die Durchschnittssignalintensität eines bestimmten Klones jeder Gruppe von OA Patienten mit der Durchschnittssignalintensität der Proben gesunden Knorpels vergleichen.

Der Expressionsspiegel eines Gens in einer bestimmten Gewebeprobe, repräsentiert durch jeweils einen bestimmten cDNA Klon, wurde in Relation zur durchschnittlichen Signalintensität eines bestimmten Klones aus dem Hybridisierungsexperiment gesetzt, indem Sonden eingesetzt wurden, die den Gewebeproben entsprachen. Eine höhere durchschnittliche Signalintensität entsprach dabei einem höheren Expressionsspiegel. Die Signifikanz der Genexpression wurde für jeden Vergleich durch Verwendung eines two-tailedT-Tests bestimmt. Ein p-Wertvon 0,1 wurde als Signifikanzschwelle eingesetzt, um so Klone zu identifizieren, die Gene repräsentieren, die in einer Gruppe von Proben aus OA Patienten gegenüber normal-Knorpel Proben differentiell exprimiert waren.

### Beispiel 5:

### Hybridisierung der cDNA Arrays

Zur Bestätigung der differentiellen Genexpression wurden einzelne, durch o.g. Vorgehen identifizierte Klone als Sonden eingesetzt und mit Arrays hybridisiert, auf denen primäre cDNA Bibliotheken von osteoarthrotischem und normalem Gewebe immobilisiert waren. Die primären cDNA Bibliotheken wurden dabei nach Standardverfahren generiert, in Bakterien eingebracht, eine Auswahl von Bakterienklonen isoliert, die die Bibliothek repräsentiert und diese Auswahl an Klonen in Mikrotiterplatten nach Standardmethoden eingelagert (siehe Bancroft et al., 1999). (1999; Methods in Microbiology 28: 67-82). Die Arrays wurden nach oben beschriebener Methodik unter Verwendung oben beschriebener Robotersysteme hergestellt, waren jedoch jeweils 23 x 23 cm groß und enthielten ca. - 40 000 PCR Produkte, die in Duplikaten aufgetragenen primären cDNA Klonen entsprachen. Für diese Experimente wurden zwei Sätze an Arrays eingesetzt; ein erster Satz von 5 Arrays entsprechend 200.000 cDNA Klonen einer cDNA Bibliothek aus normalem Gewebe und ein zweiter Satz von 5 Arrays entsprechend 200 000 cDNA Klonen einer cDNA Bibliothek aus osteoarthrotischem Gewebe.

Die PCR Produkte individueller Klone, die differentiell exprimierte Gene repräsentieren wurden durch Random Priming (DecaPrime system, Ambion) nach Standardmethoden radioaktiv markiert. Jeder markierte Klon wurde auf ein Replikat des ersten und zweiten der beiden Array Sets hybridisiert, was eine Gesamtzahl von 10 Arrays ergab, die jeweils 200.000 verschiedene primäre cDNA Klone von normalem und osteoarthrotischem Knorpelgewebe repräsentierten. Die Hybridisierungen wurden dabei in Plastiksschalen von 24 x 24 cm Fläche unter Verwendung Church Puffer (7 % SDS, 0.5 M Na-Phosphat Puffer, pH 7.0, 1 mM EDTA) bei 65°C für 16 durchgeführt. Die Waschschritte entsprachen den vorstehend beschriebenen. Nachdem die Phosphor Storage Screens den hybridisierten, in Folie verpackten Arrays über Nacht ausgesetzt waren, wurden die Arrays entsprechend obigem Vorgehen unter Verwendung desFUJI BAS 1800 II Systems bei einer Auflösung von of 100 µm gescannt und die Signale in speicherbare Daten umgewandelt. Die Anzahl der primären cDNA Klone auf jedem Satz Arrays, die mit den markierten Klonen hybridisierte wurde unter Verwendung vorstehend genannten Bildauswertungssystems und vorstehend beschriebener Vorgehensweise bestimmt. Ein bestimmtes Gen, das in einem ersten Gewebe höher exprimiert war als in einem zweiten, ergab dabei eine höhere Anzahl an Hybridisierungssignalen auf dem Array Satz, der der primären cDNA Bibliothek des ersten Gewebes entsprach als auf dem Array Satz, der die Bibliothek des zweiten Gewebes enthielt.

### Beispiel 6

Die Untersuchung der molekularen Mechanismen der OA durch vergleichende Genexpressionsanalysen stellt einen attraktiven Ansatz zur Analyse des Krankheitsbildes und der Identifizierung neuer molekularer Angriffspunkte zur pharmakologischen Intervention dar. Die Interpretation der dafür aus "Genomics-Analysen" herangezogenen Ergebnisse ist kompliziert, da in OA-erkranktem Knorpel multiple und teils gegenläufige Prozesse parallel zueinander ablaufen. Als Beispiel sei hier das gleichzeitige Auftreten von Knorpel aufbauenden und Knorpel degradierenden Aktivitäten genannt.

Um eine erste Annäherung an die potentielle Funktion eines in OA gegenüber normalem Knorpel differenziell exprimierten Gens zu erhalten, werden Systeme benötigt, in denen Knorpel degradierende und aufbauende Aktivitäten gesondert darstellbar sind.

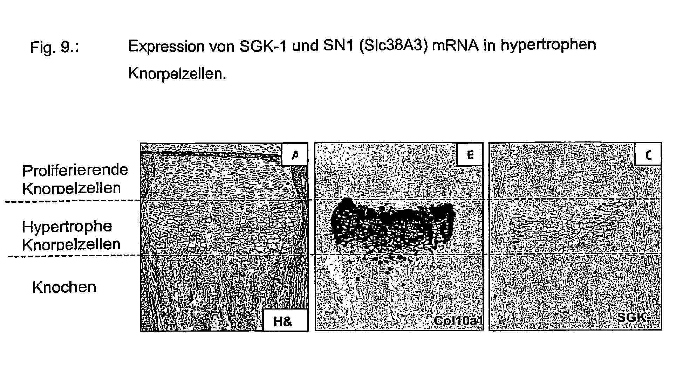

Beispiel eines dafür geeigneten Systems ist ein Skelettentwicklungs-Modell, die endochondrale Ossifikation. Während der endochondralen Ossifikation finden ebenfalls gleichzeitig Knorpelsynthese und Knorpelabbau statt. Im Gegensatz zur OA geschieht dies jedoch in klar voneinander abgegrenzten Zonen, die auch unterschiedlich differenzierte Zelltypen besitzen Gegen die Zone der proliferierenden Knorpelzellen grenzt sich der Bereich der sogenannten hypertrophen Knorpelzellen ab, die morphologisch deutlich an ihrem vergrößerten Zellvolumen erkennbar sind. In diesem Bereich finden bereits Umlagerungen und Abbauereignisse der normalen extrazellulären Knorpelmatrix (ECM) statt, deren Hauptbestandteile typischerWeise aus Kollagenen vom Typ II, IX und XI und dem Proteoglykan Aggrekan zusammengesetzt sind. Hypertrophe Knorpelzellen synthetisieren dagegen das für diese Zone spezifische Kollagen vom Typ X.In der hypertrophen Knorpelzone erfolgt eine Kalzifizierung des Gewebes, es kommt zum Verlust des anti-angiogenetische Status des Knorpels und zur Vaskularisierung des Gewebes mit nachfolgender Bildung des Knochens, der an die hypertrophe Knorpelzone der Skelettanlage angrenzt (Figur 9A). Zum frühen Entwicklungsstadium befinden sich am Ort der Knochenbildung noch Reste von Knorpel-ECM, die dann weiter abgebaut werden oder als Differenzierungsmatrix für andere Zellen dienen.

Zur Abbildung der Aktivität von Genen, die unter osteoarthrotischen Bendingungen im artikulären Knorpel exprimiert werden, an einem derartigen Skelettentwicklungsmodell, ist demnach bei einer Expression des zu untersuchenden Gens in der proliferierenden Zone eine Knorpel-aufbauende und bei Expression in der hypertrophen Zone oder dem embryonalen Knochen eine Knorpel-degradierende Funktion zugrunde zu legen.

Da für SGK-1 bisher keinerlei Expressionsdaten in Knorpelgewebe bekannt waren, wurde die Expression der SGK-1 mRNA, wie oben beschrieben, während der endochondralen Ossifikation getestet. Das Ziel dabei war es, eine erste Aussage darüber zu bekommen, welche Bedeutung dem in den vorausgegangenen Array Analysen detektierten erhöhten SGK-1 mRNA-Spiegel im Knorpelgewebe zukommt. Als zu untersuchendes Gewebe wurden die Extremitäten neonataler Mäuse verwendet. Während der Embryonalentwicklung der Maus ist zu diesem Zeitpunkt das Skelettwachstum und der Vorgang der endochondralen Ossifikation noch voll im Gange.

Die Untersuchung der SGK-1 mRNA erfolgte dann mit Hilfe der Methode der in situ Hybridisierung.

Dazu wurden die Extremitäten neonataler Mäuse und Mausembryonen verschiedener Entwicklungsstadien über Nacht in 4% Paraformaldehyd (PFA),das in Phosphat gepufferter Salzlösung (PBS) gelöst wurde, fixiert und durch schrittweisen Transfer in einer aufsteigenden Reihe verschiedener Ethanol Konzentrationen entwässert (Austausch der PBS Lösung gegen Ethanol/PBS Lösungen). Die entwässerten Gewebe wurden dann in Xylol transferiert und paraffiniert. Dazu wurde zuerst in einer Xylol/Paraffin Mischung inkubiert, bevor die Gewebe dann in reines Paraffin gegeben wurden und nach abdampfen des Xylols in dem Wachs eingebettet wurden.

Für die Herstellung der Gewebeschnitte zur in situ Hybridisierung, wurden die in Paraffin eingebetteten Embryonen und Extremitäten in Sektionen von 7 µM Durchmesser zerteilt und auf Mikroskopträger transferiert. Die Schnitte wurden dann mit Digoxygenin-11-UTP markierten und SGK-1 spezifischen Antisense Ribosonden hybridisiert. Die Transkription der Riboproben erfolgte nach Standard Methoden mit T7-RNA-Polymerase (Roche) von einem SGK-1 cDNA-Fragment welches in den Plasmid Vektor pCR^{R}-BluntII-TOPO^{R} (Invitrogen) kloniert war. Vor der in vitro Transkription wurde das Plasmid am Ende der SGK-1 cDNA Sequenz mit Kpnl (Invitrogen) linearisiert um die Transkription dort zu terminieren.

Die Sequenz des verwendeten cDNA-Fragments der murinen SGK-1 (Nukleotide 746 bis 1425) stellt eine Teilsequenz der in der NCBI-Nucleotide-Database unter BC005720 offenbarten Sequenz dar (Nukleotid 746-1425) und ist als der SEQ ID No. 13 in Figur 8 abgebildet.

Die in situ Hybridisierung der Gewebeschnitte wurde wie bei Dietz et al. (1999) beschrieben durchgeführt. Nach Detektion der Hybridisierungsprodukte wurden die Schnitte mit Deckgläschen in Kaiser's Glycerin Gelatine (Merck) eingedeckt und unter Verwendung eines Zeiss Axioplan 2 Mikroskops analysiert und photografiert. Anhand der vorstehend beschriebenen in situ Hybridisierungen konnten die Erfinder erstmals die SGK-1 mRNA in Knorpelzellen nachweisen. Die dabei nachgewiesene Expressions Domäne der SGK-1 mRNA befand sich spezifisch in den hypertrophen Knorpelzellen (Figur 9C) und war in den proliferierenden Knorpel-aufbauenden Zellen nicht detektierbar. Dieser Befund konnte eindeutig durch Vergleich mit dem Auftreten der mRNA für Kollagen X, dem Markergen für hypertrophe Knorpelzellen, gezeigt werden (Figur 9B). Der proliferierende Knorpel hingegen war eindeutig negativ in Bezug auf die SGK-1 mRNA (Figur 9C).

An den Resultaten der durchgeführten in situ Hybridisierungs Experimente zeigt sich, dass das natürliche Vorkommen von SGK-1 im Knorpel nicht mit der Synthese und Erhaltung des Knorpels verbunden ist, sondern seine Funktion bei dessen Umwandlung (Hypertrophie) und Degradation entfaltet.

Die Expression von SGK-1 in osteoarthrotischem Knorpel ist demzufolge ein Prozess der die Pathologie der OA begünstigt oder mit verursacht. SGK-1 könnte durch seine regulatorischen Eigenschaften ein neues Schlüsselmolekül für die Induktion von frühzeitigen pathologischen Knorpelveränderungen als auch der späteren degradativen Aktivitäten im Knorpel sein. Deswegen ist SGK-1 ein sehr interessantes neues Zielmolekül zur pharmakologischen Intervention der Osteoarthrose.

### Beispiel 7

Um die Vermutungen aus den vorhergegangen Experimenten zur Funktion von SGK-1 im Knorpel zu überprüfen, wurden weitere in vitro Experimente an der chondrogenen Zelllinie ATDC5 durchgeführt.

ATDC5 ist eine klonale Zelllinie, die aus der Maus Teratokarzinom Linie AT805 gewonnen wurde und unter dem Einfluss von Insulin (10 µg/ml Kulturmedium) eine knorpelige Differenzierung einschlägt. Für das experimentelle Prozedere werden die Zellen auf Zellkulturschalen als Monolayer gehalten. Nachdem sich ein konfluenter Zellrasen gebildet hat, beginnen sich unter dem Einfluss von Insulin Kondensationen der Zellen zu bilden, die nach längerer Zeitdauer lichtbrechende Häufchen fomieren und sich in ihrer Anzahl vermehren. Diese knorpelige Differenzierung ist leicht durch Anfärbung der Knorpelproteoglykane mit Alcian Blau darstellbar (Shukunami et al., 1996). Ähnlich wie bei der endochondralen Ossifikation besitzen die Zellen weiteres Differenzierungs-Potential zur Hypertrophie und Kalzifizierung. Der Vorgang wird durch spezielle Medium- und Kohlendioxidbedingungen unterstützt (Shukunami et al., 1997). Die Kalzifizierung der Kulturen kann durch Ca²⁺-Färbung mit Alizarin Rot detektiert werden. Auf molekularer Ebene ist ein deutlicher Anstieg der mRNA für Kollagen X, dem Markergen für hypertrophe Knorpelzellen erkennbar (Shukunami et al., 1997).

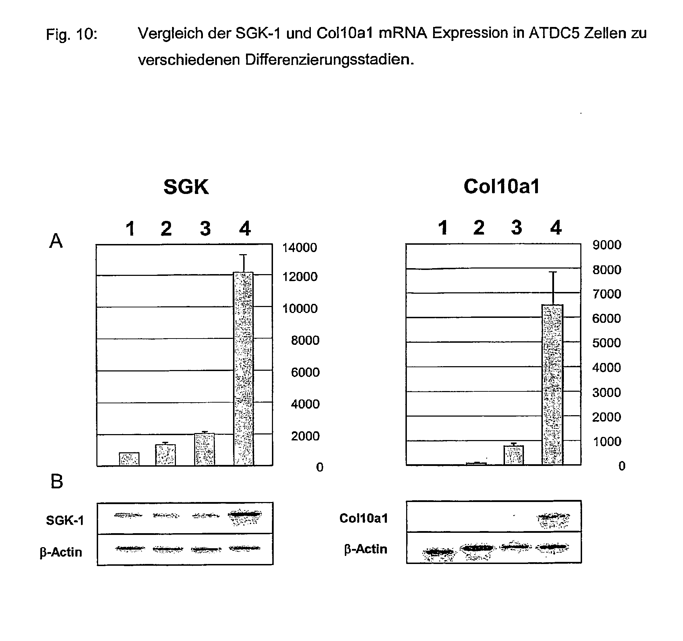

In den hier durchgeführten Experimenten, konnte die Trennung der hypertrophen Differenzierung nicht ganz eindeutig von der chondrozytär proliferierenden Aktivität der ATDC5 Zellen erzielt werden, so dass parallel zu den normalen Knorpelmarkergenen wie Typ II Kollagen auch geringe Mengen an Typ X Kollagen auf mRNA Ebene detektiert wurden. Diese stiegen jedoch dann bei der Induktion der Kalzifizierung der Kulturen deutlich an (Figur 10), während sich die Kollagen Typ II mRNA Mengen verringerten (keine Abbildung).

Die Expression der SGK-1 mRNA unter diesen Bedingungen war ähnlich der Kollagen X (Col10a1) mRNA. Während der knorpeligen Differenzierung und Proliferation der ATDC5 Zellen waren basale SGK-1 mRNA Mengen detektierbar, die dann drastisch nach Induktion der Zellen zur Hypertrophie und Kalzifizierung anstiegen (Figur 10). Zum Nachweis der mRNA Mengen von SGK-1 und Col10a1 wurden quantitative PCR-Experimente und Northern Blot Hybridisierungen durchgeführt. Beide Analyse Methoden lieferten vergleichbare Resultate. Damit wurden die Befunde aus den in situ Hybridisierungen in einem vergleichbaren in vitro Modell bestätigt.

### Beispiel 8

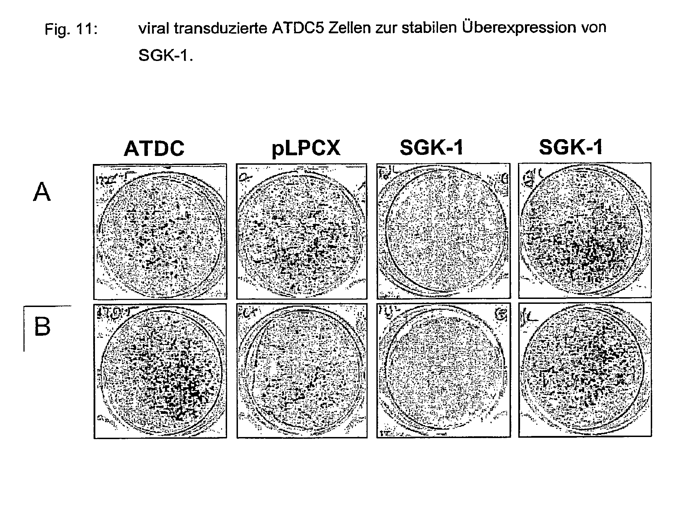

Zur gezielten Untersuchung der Funktion von SGK-1 während der Knorpel-Differenzierung, wurde humanes SGK-1 in ATDC5 Zellen durch ein MMLV (Murine Moloney Leukemia Virus) abgeleitetes retrovirales Vektor-System (pLPCX-Vektor) überexprimiert. Dabei wurden zwei verschiedene SGK-1 Varianten verwendet: eine Kinase defiziente Mutante (Mutation Threonin²⁵⁶ und Serin⁴²² zu Alanin = SGK-1 KD) und die natürlich vorkommende funktionsfähige Form (SGK-1 wt). Als Kontrolle der viralen SGK-1 Transduktion wurden ATDC5 Zellen verwendet, die nur den retroviralen Vektor ohne SGK-1 enthielten, oder untransduziert waren. Die Zellen wurden dann mit Insulin zur Knorpel Differenzierung stimuliert und nach einem Zeitraum von 21 Tagen analysiert. Das Ausmass an knorpelig differenzierten Zellen wurde durch Analyse der Proteoglykan Synthese untersucht. Dazu wurden die Kulturen mit Alcian Blau gefärbt und auf die Expression der mRNA für Aggrekan, dem hauptsächlichen Proteoglykan des Knorpels, getestet (Figur 11).

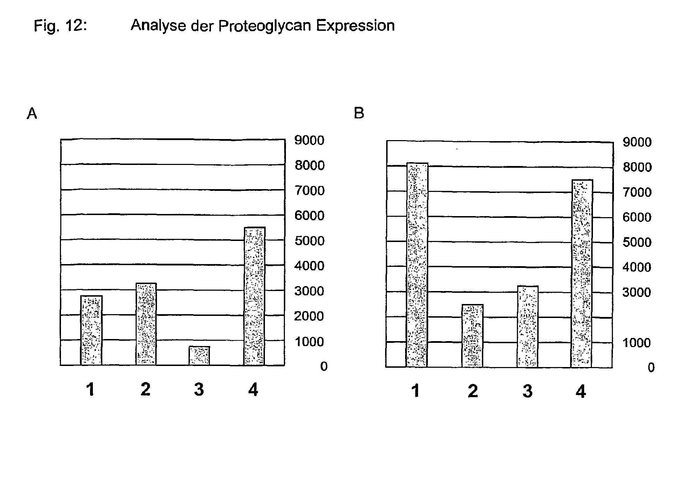

In diesen Experimenten konnte deutlich gezeigt werden, dass eine Überexpression von SGK-1 eine Inhibition der Knorpelsynthese bewirkt. Sowohl die Menge an Alcian Blau gefärbten Proteoglycan (Figur 11) als auch an Aggrekan mRNA (Figur 12) war signifikant reduziert. Die Kinase defiziente SGK-1 Form hingegen hatte keinerlei negativen Effekt auf diese Parameter (Figur 11, 12). Die beschriebenen Kontrollen verhielten sich ähnlich neutral. Dadurch konnte gezeigt werden, dass die Inhibition der Knorpelsynthese und ECM Bildung auf die Wirkung von SGK-1 zurückzuführen ist und nicht durch die artifiziell erhöhte Menge an Protein als Folge der Überexpression erzeugt wurde.

Bezüglich der Wirkung von SGK-1 in OA erkranktem artikulären Knorpel lassen sich aus diesen Experimenten verschiedene Schlüsse ziehen.

Einerseits werden SGK-1 exprimierende Knorpelzellen nicht mehr in der Lage sein, ausreichende extrazelluläre Matrix wie z.B. Proteoglykane zu synthetisieren, die essentiell für die Funktion des Gewebes sind. Zum anderen werden die Knorpelzellen gehindert Degradations-Prozesse durch Erhöhung der Expression von Genen wie Aggrekan auszugleichen oder zu reparieren. Damit ist eine Funktion von SGK-1 als potentieller Auslöser und zentraler Faktor der OA Pathologie bestätigt.

SGK-1 stellt somit ein hochinteressantes Zielmolekuel zur Findung und Entwicklung neuartiger Wirkstoffe zur Behandlung degenerativer Knorpelveränderungen, insbesondere der Osteoarthrose, dar.

Für die weitere Analyse der Funktion von SGK-1, -2, und -3 sollte eine Expression der Moleküle in artikulären Knorpelzellen wichtige Informationen liefern. Dazu können z. B. transgene und knockout Modelle der Maus nach dem Fachmann bekannten Standardmethoden (s. z.B. die aufgelistete Literatur zu Standardmethoden) hergestellt und analysiert werden.

### Beschreibung der Figuren

FIGUR 1
   Kodierende Polynukleotidsequenz der humanen Serum- Glucocorticoid Regulierten Kinase 1 (SGK1) gemäß NCBI Zugangsnummer NM_005627, AF153609 (SEQ ID No.1).
FIGUR 2
   Kodierende Polynukleotidsequenz der humanen Serum- Glucocorticoid Regulierten Kinase 2 (SGK2) gemäß NCBI Zugangsnummer AF169034, AF186470 (SEQ ID No.2).
FIGUR 3
   Kodierende Polynukleotidsequenz der humanen Serum- Glucocorticoid Regulierten Kinase 3 (SGK3) gemäß NCBI Zugangsnummer NM_013257, AF085233, AF169035 (SEQ ID No.3).
FIGUR 4
   Aminosäurensequenz der humanen Serum- Glucocorticoid Regulierten Kinase 1 (SGK1) gemäß NCBI Zugangsnummer NP_005618 (SEQ ID No.4).
FIGUR 5
   Aminosäurensequenz der humanen Serum- Glucocorticoid Regulierten Kinase 2 (SGK2) gemäß NCBI Zugangsnummer NP_733794, NP_057360 (SEQ ID No.5). In der Figur sind die ersten Aminosäuren der beiden verschiedenen SGK 2 Isoformen α und β fett markiert. Die DDB/EMBL/GenBank Zugangsnummern dieser beiden Subtypen sind AF169034 (α) und AF186470 (β). SGK2α ist ein Protein von 367 Aminosäuren, SGK2β ist dagegen ein Protein von 427 Aminosäuren.
FIGUR 6
   Aminosäurensequenz der humanen Serum- Glucocorticoid Regulierten Kinase 3 (SGK3) gemäß NCBI Zugangsnummer Q96BR1 (SEQ ID No.6). SGK3 ist ein Protein mit 429 Aminosäuren Länge.
FIGUR 7
   Aminosäuren- und kodierende Polynukleotidsequenzen funktioneller Fragmente der SGK Subtypen 1 bis 3: Figur 7A zeigt ein die Kinasedomäne enthaltendes Fragment der SGK1 kodierenden Polynukleotidsequenz (SEQ ID No.7); Figur 7B zeigt ein die Kinasedomäne enthaltendes Fragment der SGK2 kodierenden Polynukleotidsequenz (SEQ ID No.8); Figur 7C zeigt ein die Kinasedomäne enthaltendes Fragment der SGK3 kodierenden Polynukleotidsequenz (SEQ ID No.9); Figur 7D zeigt ein die Kinasedomäne enthaltendes Fragment der SGK1 Aminosäuresequenz (SEQ ID No.10); Figur 7E zeigt ein die Kinasedomäne enthaltendes Fragment der SGK3 Aminosäuresequenz (SEQ ID No.11); Figur 7F zeigt ein die Kinasedomäne enthaltendes Fragment der SGK3 Aminosäuresequenz (SEQ ID No.12);
FIGUR 8
   cDNA-Fragment der SGK-1 mRNA von Maus:
   Figur 8 zeigt die Nukleotidseqenz eines cDNA-Fragments das von der SGK-1 mRNA der Maus kloniert und für die Herstellung von Riboproben verwendet wurde (SEQ ID No.13).
FIGUR 9
   Detektion der SGK-1 mRNA Expression durch in situ Hybridisierung an Gewebeschnitten der Tibia einer neonatalen Maus.
   A: Kontroll- Färbung mit Hematoxilin und Eosin.
   B: Detektion der Col10a1 Expression in hypertrophen Knorpelzellen durch in situ Hybridisierung mit Digoxigenin markierten antisense Riboproben und anschliessender Farbreaktion. C:Detektion der SGK-1 mRNA mRNA in hypertrophen Knorpelzellen durch in situ Hybridisierung mit Digoxigenin markierten antisense Riboproben und anschliessender Farbreaktion.
   Die Abbildungen der Figur zeigten die Detektion der SGK-1 (Figur 9C) in den hypertrophen Knorpelzellen der Tibia einer neonatalen Maus durch in situ Hybridisierung von spezifischen antisense Riboproben. Zur besseren Identifizierung der Zellen ist eine entsprechende Kontrollfärbung des Gewebeschnittes gezeigt (Figur 9A).
FIGUR 10
   Vergleich der SGK-1 und Col10a1 mRNA Expression in ATDC5 Zellen zu verschiedenen Differenzierungsstadien.
   1: ATDC5 Zellen im subkonfluenten Stadium
   2: konfluente ATDC5 Zellen
   3: chondrogen differenzierte ATDC5 Zellen nach 3-wöchiger Insulingabe
   4: chondrogen differenzierte ATDC5 Zellen wie unter 3. beschrieben nach zusätzlicher Kalzifizierung über einen Zeitraum von zwei Wochen.
Figur **10A** zeigt die Analyse mRNA Mengen von SGK-1 und Col10a1 durch quantitative PCR. Als Massangabe der Expressions Amplituden sind zahlenmässige Einheiten angegeben.
Figur **10B** zeigt den Nachweis der SGK-1 und Col10a1 mRNAs durch Northern Blot Hybridisierung.
   Zur Kalibrierung für die in den Reaktionen eingesetzten Mengen an gesamt zellulärer RNA wurde in A und B die Expression der β-Actin mRNA gemessen.
FIGUR 11
   Viral transduzierte ATDC5 Zellen zur stabilen Überexpression von SGK-1.
   ATDC5: unbehandelte Zellen als Kontrolle
   pLPCX: stabile Integration des Transduktions Vektors ohne SGK-1, ebenfalls zur Kontrolle
   SGK-1 wt: Überexpression von funktioneller SGK-1
   SGK-1 KD: Überexpression einer Kinase defizienten SGK-1
   Die Zellen wurden nach Erreichen der Konfluenz 21 Tage unter chondrogenen Differenzierungs Bedingungen gehalten. Das Ausmass an chondrogener Differenzierung und Proteoglycan Produktion wurde durch Färbung mit Alcian Blau detektiert.
   SGK-1 überexprimierende Zellen zeigen signifikant reduzierte Mengen an knorpelig differenzierten Zellen und Proteoglycan Bildung, während die Kinase defiziente SGK-1 diesbezüglich keinen Wirkung hat.
   **A** und **B** zeigt den identischen Versuch in zwei unabhängigen Experimenten.
FIGUR 12
   Analyse der Proteoglycan Expression der in Figur 11 beschriebenen Proben auf der Ebene der Aggrekan mRNA durch quantitative PCR.

A und B korrespondieren mit den Proben, die in Figur11 A und B abgebildet sind.
1. ATDC5
2. pLPCX
3. SGK-1 wt
4. SGK-1 KD

Als Massangabe der Expressions Amplituden sind zahlenmässige Einheiten angegeben.Die Mengen an gemessener Aggrekan mRNA verhalten sich fast alle parallel zu den mittels Alcian Blau Färbung detektierten Proteoglycan Mengen die in Figur 11 gezeigt sind.

Nur die pLPCX Probe in Figur 11 B zeigt im Vergleich auf mRNA Ebene eine geringere Amplitude gegenüber der SGK-1 wt Probe. Eindeutig ist jedoch die stark reduzierte Aggrekan mRNA Expression nach Überexpression von SGK-1 wt im Vergleich zu ATDC5 und SGK-1 KD.

## Patentansprüche

1. Verwendung eines SGK-1-Proteins oder eines funktionellen Fragmentes davon oder einer ein solches Protein oder Fragment kodierenden Nukleinsäure für die Findung von Wirkstoffen zur Vorbeugung oder Behandlung degenerativer Knorpelveränderungen, wobei das funktionelle Fragment die Kinaseaktivität von SGK hat.

2. Verfahren zur Identifizierung von Wirkstoffen zur Vorbeugung oder Behandlung degenerativer Knorpelveränderungen mit den Schritten
a. Kontaktierung eines SGK-1-Proteins oder eines funktionellen Fragments davon, mit einem potentiellen Wirkstoff;
b. Bestimmung der SGK-1 Aktivität in Anwesenheit des potentiellen Wirkstoffs;
c. Bestimmung der SGK-1 Aktivität in Abwesenheit des potentiellen Wirkstoffs;
d. Vergleich der SGK-1 Aktivität nach b mit der nach c),
wobei das funktionelle Fragment die Kinaseaktivität von SGK hat.

3. Verfahren zur Identifizierung von Wirkstoffen zur Vorbeugung oder Behandlung degenerativer Knorpelveränderungen mit den Schritten
a. Kontaktierung einer ein SGK-1-Protein oder funktionelles Fragment davon kodierenden Nukleinsäure mit einem potentiellen Wirkstoff in einem System, das zumindest zur Transkription fähig ist;
b. Bestimmung der Menge an SGK-1 Protein oder Fragment oder der diese kodierende mRNA in Anwesenheit des potentiellen Wirkstoffs;
c. Bestimmung der Menge an SGK-1 Protein oder Fragment oder der diese kodierende mRNA in Abwesenheit des potentiellen Wirkstoffs;
d. Vergleich der Protein oder mRNA Mengen nach b) und nach c),
wobei das funktionelle Fragment die Kinaseaktivität von SGK hat.

4. Verfahren zur Identifizierung von Wirkstoffen zur Vorbeugung oder Behandlung degenerativer Knorpelveränderungen mit den Schritten
a. Kontaktierung eines SGK-1-Proteins oder eines funktionellen Fragments davon oder einer diese kodierende Nukleinsäure mit einem potentiellen Wirkstoff in einem System, dass zumindest zur Translation fähig ist;
b. Bestimmung der Aktivität des SGK-1-Proteins oder eines funktionellen Fragments davon in Anwesenheit des potentiellen Wirkstoffs;
c. Bestimmung der Aktivität des SGK-1 Proteins oder funktionellen Fragments davon in Abwesenheit des potentiellen Wirkstoffs,
wobei das funktionelle Fragment die Kinaseaktivität von SGK hat.

5. Verfahren zur Identifizierung degenerativer Knorpelveränderungen, enthaltend den Nachweis von SGK-1 in einer Knorpelgewebsprobe.

6. Verfahren zur Identifizierung degenerativer Knorpelveränderungen gemäß Anspruch 5 mit den Schritten
a. Messung des Gehaltes an SGK-1-Protein oder mRNA einer zu untersuchenden isolierten Knorpelprobe;
b. Vergleich mit dem Gehalt an SGK-1-Protein oder mRNA einer isolierten Probe von nicht degeneriertem Knorpel.

7. Verwendung gemäß Anspruch 1, Verfahren gemäß einem der Ansprüche 2 bis 4, **dadurch gekennzeichnet, dass** SGK-1 als isoliertes Molekül eingesetzt wird.

8. Verwendung gemäß Anspruch 1, **dadurch gekennzeichnet, dass** SGK-1 in einem biochemischen oder zellulären Assay eingesetzt wird.

9. Verfahren gemäß einem der Ansprüche 2 bis 4, **dadurch gekennzeichnet, dass** der Assay ein biochemischer oder zellulärer Assay ist.

10. Verwendung gemäß Anspruch 1, Verfahren gemäß einem der Ansprüche 2 bis 4, **dadurch gekennzeichnet, dass** die SGK-1 humane SGK-1 ist.

11. Verwendung gemäß Anspruch 1, **dadurch gekennzeichnet, dass** SGK-1 oder das Fragment davon ein Polynukleotid ist.

12. Verwendung gemäß Anspruch 11 oder Verfahren nach einem der Ansprüche 3 oder 4, **dadurch gekennzeichnet, dass** das Polynukleotid eine der folgenden Sequenzen enthält oder daraus besteht:
a. SEQ ID No.1;
b. eine Sequenz, die mit der Sequenz nach a) unter stringenten Bedingungen hybridisiert und für ein Polypeptid mit SGK-Funktion kodiert;
c. eine Sequenz, die aufgrund des genetischen Kodes gegenüber einer der Sequenzen nach a oder b degenieriert ist und für ein Polypeptid mit SGK-Funktion kodiert;
d. ein Fragment einer der Sequenzen nach a), b) oder c), die für ein Polypeptid mit SGK-Funktion kodiert, vorzugsweise ein Fragment mit der Sequenz gemäß SEQ ID No. 7;
e. eine, ausgehend von einer der vorstehenden Sequenzen a), b) c) oder d) durch Austausch einer oder mehrerer Basen erzeugte Sequenz, wobei der Basenaustausch nicht oder nicht ausschließlich aufgrund der Degeneriertheit des genetischen Codes erfolgt ist, und die für ein Polypeptid mit SGK-1-Funktion kodiert, wobei die Sequenz mit der Sequenz nach a) unter stringenten Bedingungen hybridisiert,
wobei das funktionelle Fragment die Kinaseaktivität von SGK hat und wobei die SGK-Funktion die Kinaseaktivität von SGK ist.

13. Verwendung gemäß Anspruch 1, **dadurch gekennzeichnet, dass** SGK-1 oder das funktionelle Fragment davon ein Polypeptid ist.

14. Verwendung gemäß Anspruch 13 oder Verfahren gemäß einem der Ansprüche 2 oder 4, **dadurch gekennzeichnet, dass** das Polypeptid kodiert wird durch ein Polynukleotid mit einer der folgenden Sequenzen:
a. SEQ ID No.1;
b. eine Sequenz, die mit mindestens einer der Sequenzen nach a) unter stringenten Bedingungen hybridisiert und für ein Polypeptid mit SGK-Funktion kodiert;
c. eine Sequenz, die aufgrund des genetischen Kodes gegenüber einer der Sequenzen nach a oder b degeneriert ist und für ein Polypeptid mit SGK-Funktion kodiert;
d. ein Fragment einer der Sequenzen nach a), b) oder c), die für ein Polypeptid mit SGK-Funktion kodiert, vorzugsweise ein Fragment mit der Sequenz gemäß SEQ ID No. 10;
e. eine, ausgehend von einer der vorstehenden Sequenzen a), b) c) oder d) durch Austausch einer oder mehrerer Basen erzeugte Sequenz, wobei der Basenaustausch nicht oder nicht ausschließlich aufgrund der Degeneriertheit des genetischen Codes erfolgt ist, und die für ein Polypeptid mit SGK-1-Funktion kodiert, wobei die Sequenz mit der Sequenz nach a) unter stringenten Bedingungen hybridisiert,
wobei das funktionelle Fragment die Kinaseaktivität von SGK hat und wobei die SGK-Funktion die Kinaseaktivität von SGK ist.

15. Verwendung oder Hochdurchsatzscreen gemäß Anspruch 13 oder Verfahren gemäß einem der Ansprüche 2 oder 4, **dadurch gekennzeichnet dass** das Polypeptid die Sequenz gemäß SEQ ID No. 4 enthält oder daraus besteht.

16. Verwendung gemäß Anspruch 13 oder Verfahren gemäß einem der Ansprüche 2 oder 4, **dadurch gekennzeichnet, dass** das funktionelle Fragment die Sequenz gemäß SEQ ID No. 10 enthält oder daraus besteht.

17. Verfahren oder Verwendung nach einem der vorstehenden Ansprüche, **dadurch gekennzeichnet, dass** die degenerative Knorpelveränderung eine arthritische Erkrankung, vorzugsweise Rheumatoide Arthritis oder Osteoarthrose und besonders bevorzugt Osteoarthrose ist.

## Claims

1. The use of an SGK-1 protein or of a functional fragment thereof or of a nucleic acid encoding such a protein or fragment for finding active ingredients for the prevention or treatment of degenerative cartilage changes, where the functional fragment has the kinase activity of SGK.

2. A method for identifying active ingredients for the prevention or treatment of degenerative cartilage changes with the steps
a. Contacting an SGK-1 protein or a functional fragment thereof with a potential active ingredient;
b. Determining the SGK-1 activity in the presence of the potential active ingredient;
c. Determining the SGK-1 activity in the absence of the potential active ingredient;
d. Comparing the SGK-1 activity from b with that from c),
where the functional fragment has the kinase activity of SGK.

3. A method for identifying active ingredients for the prevention or treatment of degenerative cartilage changes with the steps
a. Contacting a nucleic acid encoding an SGK-1 protein or functional fragment thereof with a potential active ingredient in a system which is capable at least of transcription;
b. Determining the amount of SGK-1 protein or fragment or of the mRNA encoding the latter, in the presence of the potential active ingredient;
c. Determining the amount of SGK-1 protein or fragment or of the mRNA encoding the latter, in the absence of the potential active ingredient;
d. Comparing the protein or mRNA amounts from b) and from c),
where the functional fragment has the kinase activity of SGK.

4. A method for identifying active ingredients for the prevention or treatment of degenerative cartilage changes with the steps
a. Contacting an SGK-1 protein or a functional fragment thereof or a nucleic acid coding therefor, with a potential active ingredient in a system which is capable at least of translation;
b. Determining the activity of the SGK-1 protein or a functional fragment thereof in the presence of the potential active ingredient;
c. Determining the activity of the SGK-1 protein or functional fragment thereof in the absence of the potential active ingredient,
where the functional fragment has the kinase activity of SGK.

5. A method for identifying degenerative cartilage changes comprising the detection of SGK-1 in a cartilage tissue sample.

6. A method for identifying degenerative cartilage changes as claimed in claim 5 with the steps
a. Measuring the content of SGK-1 protein or mRNA of an isolated cartilage sample to be investigated;
b. Comparing with the content of SGK-1 protein or mRNA of an isolated sample of nondegenerated cartilage.

7. The use as claimed in claim 1, method as claimed in any of claims 2 to 4, wherein SGK-1 is employed as isolated molecule.

8. The use as claimed in claim 1, wherein SGK-1 is employed in a biochemical or cellular assay.

9. The method as claimed in any of claims 2 to 4, wherein the assay is a biochemical or cellular assay.

10. The use as claimed in claim 1, method as claimed in any of claims 2 to 4, wherein SGK-1 is human SGK-1.

11. The use as claimed in claim 1, wherein SGK-1 or the fragment thereof is a polynucleotide.

12. The use as claimed in claim 11 or method as claimed in either of claims 3 or 4, wherein the polynucleotide comprises one of the following sequences or consists thereof:
a. SEQ ID No. 1;
b. a sequence which hybridizes with the sequence according to a) under stringent conditions and codes for a polypeptide having SGK function;
c. a sequence which, on the basis of the genetic code, is degenerate in relation to one of the sequences according to a or b and codes for a polypeptide having SGK function;
d. a fragment of one of the sequences according to a), b) or c) which codes for a polypeptide having SGK function, preferably a fragment having the sequence shown in SEQ ID No. 7;
e. a sequence generated starting from one of the preceding sequences a), b), c) or d) by exchange of one or more bases, where the base exchange does not, or does not exclusively, take place on the basis of the degeneracy of the genetic code, and which codes for a polypeptide having SGK-1 function, where the sequence hybridizes with the sequence according to a) under stringent conditions,
where the functional fragment has the kinase activity of SGK and where the SGK function is the kinase activity of SGK.

13. The use as claimed in claim 1, wherein SGK-1 or the functional fragment thereof is a polypeptide.

14. The use as claimed in claim 13 or method as claimed in either of claims 2 or 4, wherein the polypeptide in encoded by a polynucleotide having one of the following sequences:
a. SEQ ID No. 1;
b. a sequence which hybridizes with at least one of the sequences according to a) under stringent conditions and codes for a polypeptide having SGK function;
c. a sequence which, on the basis of the genetic code, is degenerate in relation to one of the sequences according to a or b and codes for a polypeptide having SGK function;
d. a fragment of one of the sequences according to a), b) or c) which codes for a polypeptide having SGK function, preferably a fragment having the sequence shown in SEQ ID No. 10;
e. a sequence generated starting from one of the preceding sequences a), b), c) or d) by exchange of one or more bases, where the base exchange does not, or does not exclusively, take place on the basis of the degeneracy of the genetic code, and which codes for a polypeptide having SGK-1 function, where the sequence hybridizes with the sequence according to a) under stringent conditions,
where the functional fragment has the kinase activity of SGK and where the SGK function is the kinase activity of SGK.

15. The use as claimed in claim 13 or method as claimed in either of claims 2 or 4, wherein the polypeptide comprises the sequence shown in SEQ ID No. 4 or consists thereof.

16. The use as claimed in claim 13 or method as claimed in either of claims 2 or 4, wherein the functional fragment comprises the sequence shown in SEQ ID No. 10 or consists thereof.

17. The method or use as claimed in any of the preceding claims, wherein the degenerative cartilage change is an arthritic disorder, preferably rheumatoid arthritis or osteoarthrosis and particularly preferably osteoarthrosis.

## Revendications

1. Utilisation d'une protéine SGK-1 ou d'un fragment fonctionnel de cette dernière ou d'un acide nucléique codant pour une telle protéine ou un tel fragment, pour la découverte de principes actifs destinés à la prévention ou au traitement d'altérations dégénératives du cartilage, le fragment fonctionnel ayant l'activité de kinase de la SGK.

2. Procédé pour l'identification de principes actifs destinés à la prévention ou au traitement d'altérations dégénératives du cartilage, comportant les étapes suivantes :
a. mise en contact d'une protéine SGK-1 ou d'un fragment fonctionnel de cette dernière avec un principe actif potentiel ;
b. détermination de l'activité de la SGK-1 en présence du principe actif potentiel ;
c. détermination de l'activité de la SGK-1 en l'absence du principe actif potentiel ;
d. comparaison de l'activité de la SGK-1 selon b) et selon c),
le fragment fonctionnel ayant l'activité de kinase de la SGK.

3. Procédé pour l'identification de principes actifs destinés à la prévention ou au traitement d'altérations dégénératives du cartilage, comportant les étapes suivantes :
a. mise en contact d'un acide nucléique codant pour une protéine SGK-1 ou un fragment fonctionnel de cette dernière avec un principe actif potentiel dans un système qui a au moins une capacité de transcription ;
b. détermination de la quantité de la protéine SGK-1 ou du fragment ou de l'ARNm codant pour elle, en présence du principe actif potentiel ;
c. détermination de la quantité de la protéine SGK-1 ou du fragment ou de l'ARNm codant pour cette dernière en l'absence du principe actif potentiel ;
d. comparaison des quantités de la protéine ou de l'ARNm selon b) et selon c),
le fragment fonctionnel ayant l'activité de kinase de la SGK.

4. Procédé pour l'identification de principes actifs destinés à la prévention ou au traitement d'altérations dégénératives du cartilage, comportant les étapes suivantes :
a. mise en contact d'une protéine SGK-1 ou d'un fragment fonctionnel de cette dernière ou d'un acide nucléique codant pour elle avec un principe actif potentiel dans un système qui a au moins une capacité de traduction ;
b. détermination de l'activité de la protéine SGK-1 ou d'un fragment fonctionnel de cette dernière en présence du principe actif fonctionnel ;
c. détermination de l'activité de la protéine SGK-1 ou du fragment fonctionnel de cette dernière en l'absence du principe actif potentiel,
le fragment fonctionnel ayant l'activité de kinase de la SGK.

5. Procédé pour l'identification d'altérations dégénératives du cartilage, contenant la détection de la SGK-1 dans un échantillon de tissu cartilagineux.

6. Procédé pour l'identification d'altérations dégénératives du cartilage selon la revendication 5, comportant les étapes suivantes :
a. mesure de la teneur en la protéine SGK-1 ou en l'ARNm d'un échantillon de cartilage isolé à étudier ;
b. comparaison avec la teneur en protéine SGK-1 ou ARNm d'un échantillon isolé d'un cartilage non dégénéré.

7. Utilisation selon la revendication 1, procédé selon l'une des revendications 2 à 4, **caractérisés en ce que** la SGK-1 est utilisée sous forme d'une molécule isolée.

8. Utilisation selon la revendication 1, **caractérisée en ce que** la SGK-1 est utilisée dans un essai biochimique ou cellulaire.

9. Procédé selon l'une des revendications 2 à 4, **caractérisé en ce que** l'essai est un essai biochimique ou cellulaire.

10. Utilisation selon la revendication 1, procédé selon l'une des revendications 2 à 4, **caractérisés en ce que** la SGK-1 est la SGK-1 humaine.

11. Utilisation selon la revendication 1, **caractérisée en ce que** la SGK-1 ou le fragment de cette dernière est un polynucléotide.

12. Utilisation selon la revendication 11 ou procédé selon l'une des revendications 3 ou 4, **caractérisé en ce que** le polynucléotide contient l'une des séquences suivantes, ou en est constitué :
a. SEQ ID NO:1 ;
b. une séquence qui s'hybride à la séquence selon a) dans des conditions stringentes et code pour un polypeptide ayant une fonction de la SGK ;
c. une séquence qui est dégénérée sur la base du code génétique par rapport à l'une des séquences selon a) ou b), et code pour un polypeptide ayant une fonction de la SGK ;
d. un fragment de l'une des séquences selon a), b) ou c), qui code pour un polypeptide ayant une fonction de la SGK, de préférence un fragment ayant la séquence selon SEQ ID NO:7 ;
e. une séquence produite, à partir de l'une des séquences a), b), c) ou d) ci-dessus, par échange d'une ou plusieurs bases, l'échange de bases n'étant pas réalisé ou n'étant pas exclusivement réalisé sur la base de la dégénérescence du code génétique, et qui code pour un polypeptide ayant une fonction de la SGK-1, la séquence s'hybridant à la séquence selon a) dans des conditions stringentes,
le fragment fonctionnel ayant l'activité de kinase de la SGK, et la fonction de la SGK-1 étant l'activité de kinase de la SGK.

13. Utilisation selon la revendication 1, **caractérisée en ce que** la SGK-1 ou le fragment fonctionnel de cette dernière est un polypeptide.

14. Utilisation selon la revendication 13, ou procédé selon l'une des revendications 2 ou 4, **caractérisé en ce que** le polypeptide est codé par un polynucléotide ayant l'une des séquences suivantes :
a. SEQ ID NO:1 ;
b. une séquence qui s'hybride à au moins l'une des séquences selon a) dans des conditions stringentes, et qui code pour un polypeptide ayant une fonction de la SGK ;
c. une séquence qui est dégénérée sur la base du code génétique par comparaison avec l'une des séquences selon a) ou b), et qui code pour un polypeptide ayant une fonction de la SGK ;
d. un fragment de l'une des séquences selon a), b) ou c), qui code pour un polypeptide ayant une fonction de la SGK, de préférence un fragment ayant la séquence selon SEQ ID NO:10 ;
e. une séquence produite, en partant de l'une des séquences a), b), c) ou d) ci-dessus, par échange d'une ou plusieurs bases, l'échange des bases n'étant pas réalisé ou n'étant pas exclusivement réalisé sur la base de la dégénérescence du code génétique, et qui code pour un polypeptide ayant une fonction de la SGK-1, la séquence s'hybridant à la séquence selon a) dans des conditions stringentes,
le fragment fonctionnel ayant l'activité de kinase de la SGK, et la fonction de la SGK étant l'activité de kinase de la SGK.

15. Utilisation ou criblage à haut débit selon la revendication 13, ou procédé selon l'une des revendications 2 ou 4, **caractérisé en ce que** le polypeptide contient la séquence selon SEQ ID NO:4 ou en est constitué.

16. Utilisation selon la revendication 13 ou procédé selon l'une des revendications 2 ou 4, **caractérisé en ce que** le fragment fonctionnel contient la séquence selon SEQ ID NO:10 ou en est constitué.

17. Procédé ou utilisation selon l'une des revendications précédentes, **caractérisé en ce que** l'altération dégénérative du cartilage est une maladie arthritique, de préférence la polyarthrite rhumatoïde ou l'ostéoarthrose et d'une manière particulièrement préférée l'ostéoarthrose.


### SEQUENZPROTOKOLL

<110> Sanofi-Aventis Deutschland GmbH
<120> Verwendung der Serum-/Glucocorticoid regulierten Kinase
<130> DEAV 2004/0077
<140> 10 2004 059 781.2
   <141> 2004-12-10
<160> 13
<170> PatentIn Ver. 2.1
<210> 1
   <211> 2370
   <212> DNA
   <213> Homo sapiens
<400> 1
   cacgagggag cgctaacgtc tttctgtctc cccgcggtgg tgatgacggt gaaaactgag 60
   gctgctaagg gcaccctcac ttactccagg atgaggggca tggtggcaat tctcatcgct 120
   ttcatgaagc agaggaggat gggtctgaac gactttattc agaagattgc caataactcc 180
   tatgcatgca aacaccctga agttcagtcc atcttgaaga tctcccaacc tcaggagcct 240
   gagcttatga atgccaaccc ttctcctcca ccaagtcctt ctcagcaaat caaccttggc 300
   ccgtcgtcca atcctcatgc taaaccatct gactttcact tcttgaaagt gatcggaaag 360
   ggcagttttg gaaaggttct tctagcaaga cacaaggcag aagaagtgtt ctatgcagtc 420
   aaagttttac agaagaaagc aatcctgaaa aagaaagagg agaagcatat tatgtcggag 480
   cggaatgttc tgttgaagaa tgtgaagcac cctttcctgg tgggccttca cttctctttc 540
   cagactgctg acaaattgta ctttgtccta gactacatta atggtggaga gttgttctac 600
   catctccaga gggaacgctg cttcctggaa ccacgggctc gtttctatgc tgctgaaata 660
   gccagtgcct tgggctacct gcattcactg aacatcgttt atagagactt aaaaccagag 720
   aatattttgc tagattcaca gggacacatt gtccttactg atttcggact ctgcaaggag 780
   aacattgaac acaacagcac aacatccacc ttctgtggca cgccggagta tctcgcacct 840
   gaggtgcttc ataagcagcc ttatgacagg actgtggact ggtggtgcct gggagctgtc 900
   ttgtatgaga tgctgtatgg cctgccgcct ttttatagcc gaaacacagc tgaaatgtac 960
   gacaacattc tgaacaagcc tctccagctg aaaccaaata ttacaaattc cgcaagacac 1020
   ctcctggagg gcctcctgca gaaggacagg acaaagcggc tcggggccaa ggatgacttc 1080
   atggagatta agagtcatgt cttcttctcc ttaattaact gggatgatct cattaataag 1140
   aagattactc ccccttttaa cccaaatgtg agtgggccca acgagctacg gcactttgac 1200
   cccgagttta ccgaagagcc tgtccccaac tccattggca agtcccctga cagcgtcctc 1260
   gtcacagcca gcgtcaagga agctgccgag gctttcctag gcttttccta tgcgcctccc 1320
   acggactctt tcctctgaac cctgttaggg cttggtttta aaggatttta tgtgtgtttc 1380
   cgaatgtttt agttagcctt ttggtggagc cgccagctga caggacatct tacaagagaa 1440
   tttgcacatc tctggaagct tagcaatctt attgcacact gttcgctgga attttttgaa 1500
   gagcacattc tcctcagtga gctcatgagg ttttcatttt tattcttcct tccaacgtgg 1560
   tgctatctct gaaacgagcg ttagagtgcc gccttagacg gaggcaggag tttcgttaga 1620
   aagcggacct gttctaaaaa aggtctcctg cagatctgtc tgggctgtga tgacgaatat 1680
   tatgaaatgt gccttttctg aagagattgt gttagctcca aagcttttcc tatcgcagtg 1740
   tttcagttct ttattttccc ttgtggatat gctgtgtgaa ccgtcgtgtg agtgtggtat 1800
   gcctgatcac agatggattt tgttataagc atcaatgtga cacttgcagg acactacaac 1860
   gtgggacatt gtttgtttct tccatatttg gaagataaat ttatgtgtag acttttttgt 1920
   aagatacggt taataactaa aatttattga aatggtcttg caatgactcg tattcagatg 1980
   cctaaagaaa gcattgctgc tacaaatatt tctattttta gaaagggttt ttatggacca 2040
   atgccccagt tgtcagtcag agccgttggt gtttttcatt gtttaaaatg tcacctgtaa 2100
   aatgggcatt atttatgttt ttttttttgc attcctgata attgtatgta ttgtataaag 2160
   aacgtctgta cattgggtta taacactagt atatttaaac ttacaggctt atttgtaatg 2220
   taaaccacca ttttaatgta ctgtaattaa catggttata atacgtacaa tccttccctc 2280
   atcccatcac acaacttttt ttgtgtgtga taaactgatt ttggtttgca ataaaacctt 2340
   gaaaaataaa aaaaaaaaaa aaaaaaaaaa                                  2370
<210> 2
   <211> 2146
   <212> DNA
   <213> Homo sapiens
<400> 2
   atgggttcag actttatgcc ctgaaaagat ccttccagcc ctggccatct tggacttctg 60
   gagctaccct ggctcacagg ggtcttgttg ccctgggtgt ccccagttct tgaaaagaat 120
   cagcctggga ggggccacac cctgaccatc cccctttatc ccttctgaga tgtttgttag 180
   gaagtctggg tccaggggat atcatttctt gttccatcca tgcaggggtt gcttacctcg 240
   ggtaggaaac cctcaggcgg tggcaggtgc acaggtaggg gaggatggag agggcagtgg 300
   tgcctgaagc cctggatggg cggagctgac cccccaacac caactctatc atgcctgctc 360
   ctccctgtcc ccccagagct gcctgatcat tgctacagaa tgaactctag cccagctggg 420
   accccaagtc cacagccctc cagggccaat gggaacatca acctggggcc ttcagccaac 480
   ccaaatgccc agcccacgga cttcgacttc ctcaaagtca tcggcaaagg gaactacggg 540
   aaggtcctac tggccaagcg caagtctgat ggggcgttct atgcagtgaa ggtactacag 600
   aaaaagtcca tcttaaagaa gaaagagcag agccacatca tggcagagcg cagtgtgctt 660
   ctgaagaacg tgcggcaccc cttcctcgtg ggcctgcgct actccttcca gacacctgag 720
   aagctctact tcgtgctcga ctatgtcaac gggggagagc tcttcttcca cctgcagcgg 780
   gagcgccggt tcctggagcc ccgggccagg ttctacgctg ctgaggtggc cagcgccatt 840
   ggctacctgc actccctcaa catcatttac agggatctga aaccagagaa cattctcttg 900
   gactgccagg gacacgtggt gctgacggat tttggcctct gcaaggaagg tgtagagcct 960
   gaagacacca catccacatt ctgtggtacc cctgagtact tggcacctga agtgcttcgg 1020
   aaagagcctt atgatcgagc agtggactgg tggtgcttgg gggcagtcct ctacgagatg 1080
   ctccatggcc tgccgccctt ctacagccaa gatgtatccc agatgtatga gaacattctg 1140
   caccagccgc tacagatccc cggaggccgg acagtggccg cctgtgacct cctgcaaagc 1200
   cttctccaca aggaccagag gcagcggctg ggctccaaag cagactttct tgagattaag 1260
   aaccatgtat tcttcagccc cataaactgg gatgacctgt accacaagag gctaactcca 1320
   cccttcaacc caaatgtgac aggacctgct gacttgaagc attttgaccc agagttcacc 1380
   caggaagctg tgtccaagtc cattggctgt acccctgaca ctgtggccag cagctctggg 1440
   gcctcaagtg cattcctggg attttcttat gcgccagagg atgatgacat cttggattgc 1500
   tagaagagaa ggacctgtga aactactgag gccagctggt attagtaagg aattaccttc 1560
   agctgctagg aagagcgact caaactaaca atggcttcaa cgagaagcag gtttattttt 1620
   tccagcacat aaaagaaaaa taatgtttcg gagtccagga ctggcaggac aggtcatcag 1680
   atactcagag gctgtatctc tgccctgcca accttgacaa atggcttcca atgttaggtt 1740
   tgctacaaga tggttactgg agctctagct gcctattttg tgtttaggga agggaaaatg 1800
   gaggaaaggg gagaagagca aagggcgctt ttaaagagct ttcccaaaag ctccccccaa 1860
   tgacttttgc ttccatctca ctaaccaccc acccctacct ggaatggagg ctgggaaatg 1920
   tggcttattt gctgggtacg tgactatccc taataacaaa ggggttttga ccctaagaca 1980
   ttaggggaga atgttgggta ggcagccagc cctcttttac catagggcct cctggtgttt 2040
   ggattttgat ctcaatgtgt aaaatgacag agatgtaaca agctcatagg gtatcaatat 2100
   ctcttattgt tctatgttga aaaaaaaaaa aaaaaaaaaa aaaaaa 2146
<210> 3
   <211> 3984
   <212> DNA
   <213> Homo sapiens
<400> 3
   ctgctgactg tttcttcgga tgcatttttt ggtgtgctct tgagggatta aatgcaaaga 60
   gatcacacca tggactacaa ggaaagctgc ccaagtgtaa gcattcccag ctccgatgaa 120
   cacagagaga aaaagaagag gtttactgtt tataaagttc tggtttcagt gggaagaagt 180
   gaatggtttg tcttcaggag atatgcagag gttgataaac tttataacac tttaaaaaaa 240
   cagtttcctg ctatggccct gaagattcct gccaagagaa tatttggtga taattttgat 300
   ccagatttta ttaaacaaag acgagcagga ctaaacgaat tcattcagaa cctagttagg 360
   tatccagaac tttataacca tccagatgtc agagcattcc ttcaaatgga cagtccaaaa 420
   caccagtcag gtccatctga agatgaggat gaaagaagtt ctcagaagct acactctacc 480
   tcacagaaca tcaacctggg accgtctgga aatcctcatg ccaaaccaac tgactttgat 540
   ttcttaaaag ttattggaaa aggcagcttt ggcaaggttc ttcttgcaaa acggaaactg 600
   gatggaaaag tttatgctgt caaagtgtta cagaaaaaaa tagttctcaa cagaaaagag 660
   caaaaacata ttatggctga acgtaatgtg ctcttgaaaa atgtgaaaca tccgtttttg 720
   gttggattgc attattcctt ccaaacaact gaaaagcttt attttgttct ggattttgtt 780
   aatggagggg agcttttttt ccacttacaa agagaacggt cctttcctga gcacagagct 840
   aggttttacg ctgctgaaat tgctagtgca ttgggttact tacattccat caaaatagta 900
   tacagagact tgaaaccaga aaatattctt gtggattcag taggacatgt tgtcttaaca 960
   gattttgggc tttgtaaaga aggaattgct atttctgaca ccactaccac attttgtggg 1020
   acaccagagt atcttgcacc tgaagtaatt agaaaacagc cctatgacaa taccgtagat 1080
   tggtggtgcc ttggggctgt tctgtatgaa atgctgtatg gattgcctcc tttttattgc 1140
   cgagatgttg ctgaaatgta tgacaatatc cttcacaaac ccctaagttt gaggccagga 1200
   gtgagtctta gagcctggtc cattctggaa gaactcctag aaaaagacag gcaaaatcga 1260
   cttggtgcca aggaagactt tcttgaaatt cagaatcatc cttttttgga atcactcagc 1320
   tgggctgacc ttgtacaaaa gaagattcca ccaccattta atcctaatgt ggctggacca 1380
   gatgatatca gaaactttga cacagcattt acagaagaaa cagttccata ttctgtgtgt 1440
   gtatcttctg actattctat agtgaatgcc agtgtattgg aggcagatga tgcattcgtt 1500
   ggtttctctt atgcacctcc ttcagaagac ttatttttgt gagcagttgc catacagaaa 1560
   ccattgagca aaataagtct atagatggga ctgaaacttc tatttgtgtg aatatattca 1620
   aatatgtata actagtgcct catttttata tgtaatgatg aaaactatga aaaaatgtat 1680
   tttcttctat gtgcaagaaa aatagggcat ttcaaagagc tgttttgatt aaaatttata 1740
   ttcttgttta ataagcttat ttttaaacaa tttaaaagct attattctta gcattaacct 1800
   atttttaaag aaaccttttt gctattgact gttttttccc tctaagttta cactaacatc 1860
   tacccaagat agactgtttt ttaacagtca atttcagttc agctaacata tattaatacc 1920
   tttgtaactc tttgctatgg cttttgttat cacaccaaaa ctatgcaatt ggtacatggt 1980
   tgtttaagaa gaaaccgtat ttttccatga taaatcactg tttgaaatat ttggtcatgg 2040
   tatgatcgaa atgtaaaagc ataattaaca cattggctgc tagttaacaa ttggaataac 2100
   tttattctgc agatcattta agaagtaaca ggccgggcgc gggttgtcac gcctgtaatc 2160
   ccagcacttt gggaggctga ggcgggcaga tcacctgagg tcaggagttg gagaccagcc 2220
   tgaccaacat ggacaaaccc cgtctctact aaaaatacaa aattggcagg gtgtggtgca 2280
   catgcctata atcccagcta cttgggaggc taaggcagga gaatcgcttg aacccgggag 2340
   gcggaggttg cagtgagccg agatcgcacc attgcactcc tgcctgggca acaagagtga 2400
   aactccatct ccaaaaaaaa aaaggaaaaa gtaacaaaag gaaattattt gtttttgaaa 2460
   taccagttca actttgtgga ttatttttcc tctgaaggaa aagaaaaggc ttaatggtta 2520
   ggatttttaa gtattcccaa agatctgaag ggtaataaaa tgtactggat tttttaaggt 2580
   ggtaccaaaa atgaatgtct gtcatatatt tatattacaa atacattata tttatgttct 2640
   attcatcttt tgaatgttta gtatgctatt aagtcattct gaatctttgt atttgctttt 2700
   gcaaataggt atttcaaagc tcttttccta actggttaag taaaataaaa aattgagctt 2760
   tctagaatat ttgcctaatt gggaattaaa aagtaaaata ataggccagg catggtggct 2820
   catgcctata agcaccctgg gaggccgagg caggcagatt atttgagctc aggagtttga 2880
   gaccatcctg ggcaacatgg cgaaacccta tctctacaaa aaatacaaaa attagccaga 2940
   catggtggca catgccttta gtcccagcta ctcaggaggc tgaagttgga ggatggcttg 3000
   agcccacgag atggaagttg cagtgaactg aaattgtgcc actgcacttt tcagcctggg 3060
   tgccacagcg agaccctagt taagagaaaa aaaaagtaaa aacaaattgt gggtcaaagt 3120
   aaatgtatac agttttatta caatgtaaca aaagttgaaa atcgggcccg gaatttatcc 3180
   aatttcagat atatcttaga aaaagcacag gaaacagacc ttaaaattgt aacctaccaa 3240
   ctaacttaca tgcttataaa agtaaaggag aataactggc cgggcacggt ggctcatgcc 3300
   tataaaattc cagcactttg ggaggccaag gcaggaggat cacttgagcc tatgagttca 3360
   agaccagcct aggcaatgta gtgacacctc atttctattt attttaaaaa aaagagagag 3420
   taactacaga agaactttaa aaaataaaaa ataagcttac cttggattct tggcttagag 3480
   tagaggtttt ttttaagtta tggaggaaac atttttgtaa aagtttaatg acccacttta 3540
   gatgctccaa gaacaagcat cccttccatg tatgtcttga gaaagaaatc acagaagcat 3600
   ttctcaccaa tactctttgg cttaaaatgt tcagcagaat tgggcagtgg gggtgacttt 3660
   tcttatatta ataatattta catccaatac actgaatctt cctttagagg taagacttta 3720
   atatctatac tgtaaatatt tggtttattt ggcactactg taagttttgt ttttcacaaa 3780
   gctcttatta tgaagcaaaa taaaaattct agtttcttgt atgatttttt gtactcattc 3840
   attcctgtta agctgccaaa aattaaagtg caatattgta tatttttaag aacaaattta 3900
   aaatagaatt ttgatgtttc tcagatcaca agaaatacaa atctatatag tataataaaa 3960
   tcagcaaaag atcaaaaaaa aaaa                                        3984
<210> 4
   <211> 431
   <212> PRT
   <213> Homo sapiens
<400> 4
<210> 5
   <211> 427
   <212> PRT
   <213> Homo sapiens
<400> 5
   Met Gln Gly Leu Leu Thr Ser Gly Arg Lys Pro Ser Gly Gly Gly Arg
   1               5                   10                  15
   Cys Thr Gly Arg Gly Gly Trp Arg Gly Gln Trp Cys Leu Lys Pro Trp
               20                  25                  30
   Met Gly Gly Ala Asp Pro Pro Thr Pro Thr Leu Ser Cys Leu Leu Leu
           35                  40                  45
   Pro Val Pro Pro Glu Leu Pro Asp His Cys Tyr Arg Met Asn Ser Ser
       50                  55                  60
   Pro Ala Gly Thr Pro Ser Pro Gln Pro Ser Arg Ala Asn Gly Asn Ile
   65                  70                  75                  80
   Asn Leu Gly Pro Ser Ala Asn Pro Asn Ala Gln Pro Thr Asp Phe Asp
                   85                  90                  95
   Phe Leu Lys Val Ile Gly Lys Gly Asn Tyr Gly Lys Val Leu Leu Ala
               100                 105                 110
   Lys Arg Lys Ser Asp Gly Ala Phe Tyr Ala Val Lys Val Leu Gln Lys
           115                 120                 125
   Lys Ser Ile Leu Lys Lys Lys Glu Gln Ser His Ile Met Ala Glu Arg
       130                 135                 140
   Ser Val Leu Leu Lys Asn Val Arg His Pro Phe Leu Val Gly Leu Arg
   145                 150                 155                 160
   Tyr Ser Phe Gln Thr Pro Glu Lys Leu Tyr Phe Val Leu Asp Tyr Val
                   165                 170                 175
   Asn Gly Gly Glu Leu Phe Phe His Leu Gln Arg Glu Arg Arg Phe Leu
               180                 185                 190
   Glu Pro Arg Ala Arg Phe Tyr Ala Ala Glu Val Ala Ser Ala Ile Gly
           195                 200                 205
   Tyr Leu His Ser Leu Asn Ile Ile Tyr Arg Asp Leu Lys Pro Glu Asn
       210                 215                 220
   Ile Leu Leu Asp Cys Gln Gly His Val Val Leu Thr Asp Phe Gly Leu
   225                 230                 235                 240
   Cys Lys Glu Gly Val Glu Pro Glu Asp Thr Thr Ser Thr Phe Cys Gly
                   245                 250                 255
   Thr Pro Glu Tyr Leu Ala Pro Glu Val Leu Arg Lys Glu Pro Tyr Asp
               260                 265                 270
   Arg Ala Val Asp Trp Trp Cys Leu Gly Ala Val Leu Tyr Glu Met Leu
           275                 280                 285
   His Gly Leu Pro Pro Phe Tyr Ser Gln Asp Val Ser Gln Met Tyr Glu
       290                 295                 300
   Asn Ile Leu His Gln Pro Leu Gln Ile Pro Gly Gly Arg Thr Val Ala
   305                 310                 315                 320
   Ala Cys Asp Leu Leu Gln Ser Leu Leu His Lys Asp Gln Arg Gln Arg
                   325                 330                 335
   Leu Gly Ser Lys Ala Asp Phe Leu Glu Ile Lys Asn His Val Phe Phe
   340 345 350
   Ser Pro Ile Asn Trp Asp Asp Leu Tyr His Lys Arg Leu Thr Pro Pro
   355 360 365
   Phe Asn Pro Asn Val Thr Gly Pro Ala Asp Leu Lys His Phe Asp Pro
   370 375 380
   Glu Phe Thr Gln Glu Ala Val Ser Lys Ser Ile Gly Cys Thr Pro Asp
   385 390 395 400
   Thr Val Ala Ser Ser Ser Gly Ala Ser Ser Ala Phe Leu Gly Phe Ser
   405 410 415
   Tyr Ala Pro Glu Asp Asp Asp Ile Leu Asp Cys
   420 425
<210> 6
   <211> 496
   <212> PRT
   <213> Homo sapiens
<400> 6
   Met Gln Arg Asp His Thr Met Asp Tyr Lys Glu Ser Cys Pro Ser Val
   1 5 10 15
   Ser Ile Pro Ser Ser Asp Glu His Arg Glu Lys Lys Lys Arg Phe Thr
   20 25 30
   Val Tyr Lys Val Leu Val Ser Val Gly Arg Ser Glu Trp Phe Val Phe
   35 40 45
   Arg Arg Tyr Ala Glu Val Asp Lys Leu Tyr Asn Thr Leu Lys Lys Gln
   50 55 60
   Phe Pro Ala Met Ala Leu Lys Ile Pro Ala Lys Arg Ile Phe Gly Asp
   65 70 75 80
   Asn Phe Asp Pro Asp Phe Ile Lys Gln Arg Arg Ala Gly Leu Asn Glu
   85 90 95
   Phe Ile Gln Asn Leu Val Arg Tyr Pro Glu Leu Tyr Asn His Pro Asp
   100 105 110
   Val Arg Ala Phe Leu Gln Met Asp Ser Pro Lys His Gln Ser Gly Pro
   115 120 125
   Ser Glu Asp Glu Asp Glu Arg Ser Ser Gln Lys Leu His Ser Thr Ser
   130 135 140
   Gln Asn Ile Asn Leu Gly Pro Ser Gly Asn Pro His Ala Lys Pro Thr
   145 150 155 160
   Asp Phe Asp Phe Leu Lys Val Ile Gly Lys Gly Ser Phe Gly Lys Val
   165 170 175
   Leu Leu Ala Lys Arg Lys Leu Asp Gly Lys Val Tyr Ala Val Lys Val
   180 185 190
   Leu Gln Lys Lys Ile Val Leu Asn Arg Lys Glu Gln Lys His Ile Met
   195 200 205
   Ala Glu Arg Asn Val Leu Leu Lys Asn Val Lys His Pro Phe Leu Val
   210 215 220
   Gly Leu His Tyr Ser Phe Gln Thr Thr Glu Lys Leu Tyr Phe Val Leu
   225 230 235 240
   Asp Phe Val Asn Gly Gly Glu Leu Phe Phe His Leu Gln Arg Glu Arg
   245 250 255
   Ser Phe Pro Glu His Arg Ala Arg Phe Tyr Ala Ala Glu Ile Ala Ser
   260 265 270
   Ala Leu Gly Tyr Leu His Ser Ile Lys Ile Val Tyr Arg Asp Leu Lys
   275 280 285
   Pro Glu Asn Ile Leu Val Asp Ser Val Gly His Val Val Leu Thr Asp
   290 295 300
   Phe Gly Leu Cys Lys Glu Gly Ile Ala Ile Ser Asp Thr Thr Thr Thr
   305 310 315 320
   Phe Cys Gly Thr Pro Glu Tyr Leu Ala Pro Glu Val Ile Arg Lys Gln
   325 330 335
   Pro Tyr Asp Asn Thr Val Asp Trp Trp Cys Leu Gly Ala Val Leu Tyr
   340 345 350
   Glu Met Leu Tyr Gly Leu Pro Pro Phe Tyr Cys Arg Asp Val Ala Glu
   355 360 365
   Met Tyr Asp Asn Ile Leu His Lys Pro Leu Ser Leu Arg Pro Gly Val
   370 375 380
   Ser Leu Arg Ala Trp Ser Ile Leu Glu Glu Leu Leu Glu Lys Asp Arg
   385 390 395 400
   Gln Asn Arg Leu Gly Ala Lys Glu Asp Phe Leu Glu Ile Gln Asn His
   405 410 415
   Pro Phe Phe Glu Ser Leu Ser Trp Ala Asp Leu Val Gln Lys Lys Ile
   420 425 430
   Pro Pro Pro Phe Asn Pro Asn Val Ala Gly Pro Asp Asp Ile Arg Asn
   435 440 445
   Phe Asp Thr Ala Phe Thr Glu Glu Thr Val Pro Tyr Ser Val Cys Val
   450 455 460
   Ser Ser Asp Tyr Ser Ile Val Asn Ala Ser Val Leu Glu Ala Asp Asp
   465 470 475 480
   Ala Phe Val Gly Phe Ser Tyr Ala Pro Pro Ser Glu Asp Leu Phe Leu
   485 490 495
<210> 7
   <211> 846
   <212> DNA
   <213> Homo sapiens
<400> 7
   tttcacttct tgaaagtgat cggaaagggc agttttggaa aggttcttct agcaagacac 60
   aaggcagaag aagtgttcta tgcagtcaaa gttttacaga agaaagcaat cctgaaaaag 120
   aaagaggaga agcatattat gtcggagcgg aatgttctgt tgaagaatgt gaagcaccct 180
   ttcctggtgg gccttcactt ctctttccag actgctgaca aattgtactt tgtcctagac 240
   tacattaatg gtggagagtt gttctaccat ctccagaggg aacgctgctt cctggaacca 300
   cgggctcgtt tctatgctgc tgaaatagcc agtgccttgg gctacctgca ttcactgaac 360
   atcgtttata gagacttaaa accagagaat attttgctag attcacaggg acacattgtc 420
   cttactgact tcggactctg caaggagaac attgaacaca acagcacaac atccaccttc 480
   tgtggcacgc cggagtatct cgcacctgag gtgcttcata agcagcctta tgacaggact 540
   gtggactggt ggtgcctggg agctgtcttg tatgagatgc tgtatggcct gccgcctttt 600
   tatagccgaa acacagctga aatgtacgac aacattctga acaagcctct ccagctgaaa 660
   ccaaatatta caaattccgc aagacacctc ctggagggcc tcctgcagaa ggacaggaca 720
   aagcggctcg gggccaagga tgacttcatg gagattaaga gtcatgtctt cttctcctta 780
   attaactggg atgatctcat taataagaag attactcccc cttttaaccc aaatgtgagt 840
   gggccc                                                            846
<210> 8
   <211> 780
   <212> DNA
   <213> Homo sapiens
<400> 8
   gacttcgact tcctcaaagt catcggcaaa gggaactacg ggaaggtcct actggccaag 60
   cgcaagtctg atggggcgtt ctatgcagtg aaggtactac agaaaaagtc catcttaaag 120
   aagaaagagc agagccacat catggcagag cgcagtgtgc ttctgaagaa cgtgcggcac 180
   cccttcctcg tgggcctgcg ctactccttc cagacacctg agaagctcta cttcgtgctc 240
   gactatgtca acgggggaga gctcttcttc cacctgcagc gggagcgccg gttcctggag 300
   ccccgggcca ggttctacgc tgctgaggtg gccagcgcca ttggctacct gcactccctc 360
   aacatcattt acagggatct gaaaccagag aacattctct tggactgcca gggacacgtg 420
   gtgctgacgg attttggcct ctgcaaggaa ggtgtagagc ctgaagacac cacatccaca 480
   ttctgtggta cccctgagta cttggcacct gaagtgcttc ggaaagagcc ttatgatcga 540
   gcagtggact ggtggtgctt gggggcagtc ctctacgaga tgctccatgg cctgccgccc 600
   ttctacagcc aagatgtatc ccagatgtat gagaacattc tgcaccagcc gctacagatc 660
   cccggaggcc ggacagtggc cgcctgtgac ctcctgcaaa gccttctcca caaggaccag 720
   aggcagcggc tgggctccaa agcagacttt cttgagatta agaaccatgt attcttcagc 780
<210> 9
   <211> 780
   <212> DNA
   <213> Homo sapiens
<400> 9
   actttgattt cttaaaagtt attggaaaag gcagctttgg caaggttctt cttgcaaaac 60
   ggaaactgga tggaaaattt tatgctgtca aagtgttaca gaaaaaaata gttctcaaca 120
   gaaaagagca aaaacatatt atggctgaac gtaatgtgct cttgaaaaat gtgaaacatc 180
   cgtttttggt tggattgcat tattccttcc aaacaactga aaagctttat tttgttctgg 240
   attttgttaa tggaggggag ctttttttcc acttacaaag agaacggtcc tttcctgagc 300
   acagagctag gttttacgct gctgaaattg ctagtgcatt gggttactta cattccatca 360
   aaatagtata cagagacttg aaaccagaaa atattctttt ggattcagta ggacatgttg 420
   tcttaacaga ttttgggctt tgtaaagaag gaattgctat ttctgacacc actaccacat 480
   tttgtgggac accagagtat cttgcacctg aagtaattag aaaacagccc tatgacaata 540
   ctgtagattg gtggtgcctt ggggctgttc tgtatgaaat gctgtatgga ttgcctcctt 600
   tttattgccg agatgttgct gaaatgtatg acaatatcct tcacaaaccc ctaagtttga 660
   ggccaggagt gagtcttaca gcctggtcca ttctggaaga actcctagaa aaagacaggc 720
   aaaatcgact tggtgccaag gaagactttc ttgaaattca gaatcatcct ttttttgaat 780
<210> 10
   <211> 260
   <212> PRT
   <213> Homo sapiens
<400> 10
   Ser Asp Phe His Phe Leu Lys Val Ile Gly Lys Gly Ser Phe Gly Lys
   1               5                   10                  15
   Val Leu Leu Ala Arg His Lys Ala Glu Glu Val Phe Tyr Ala Val Lys
               20                  25                  30
   Val Leu Gln Lys Lys Ala Ile Leu Lys Lys Lys Glu Glu Lys His Ile
           35                  40                  45
   Met Ser Glu Arg Asn Val Leu Leu Lys Asn Val Lys His Pro Phe Leu
       50                  55                  60
   Val Gly Leu His Phe Ser Phe Gln Thr Ala Asp Lys Leu Tyr Phe Val
   65                  70                  75                  80
   Leu Asp Tyr Ile Asn Gly Gly Glu Leu Phe Tyr His Leu Gln Arg Glu
                   85                  90                  95
   Arg Cys Phe Leu Glu Pro Arg Ala Arg Phe Tyr Ala Ala Glu Ile Ala
               100                 105                 110
   Ser Ala Leu Gly Tyr Leu His Ser Leu Asn Ile Val Tyr Arg Asp Leu
           115                 120                 125
   Lys Pro Glu Asn Ile Leu Leu Asp Ser Gln Gly His Ile Val Leu Thr
       130                 135                 140
   Asp Phe Gly Leu Cys Lys Glu Asn Ile Glu His Asn Ser Thr Thr Ser
   145                 150                 155                 160
   Thr Phe Cys Gly Thr Pro Glu Tyr Leu Ala Pro Glu Val Leu His Lys
                   165                 170                 175
   Gln Pro Tyr Asp Arg Thr Val Asp Trp Trp Cys Leu Gly Ala Val Leu
               180                 185                 190
   Tyr Glu Met Leu Tyr Gly Leu Pro Pro Phe Tyr Ser Arg Asn Thr Ala
           195                 200                 205
   Glu Met Tyr Asp Asn Ile Leu Asn Lys Pro Leu Gln Leu Lys Pro Asn
       210                 215                 220
   Ile Thr Asn Ser Ala Arg His Leu Leu Glu Gly Leu Leu Gln Lys Asp
   225                 230                 235                 240
   Arg Thr Lys Arg Leu Gly Ala Lys Asp Asp Phe Met Glu Ile Lys Ser
                   245                 250                 255
   His Val Phe Phe
               260
<210> 11
   <211> 260
   <212> PRT
   <213> Homo sapiens
<400> 11
   Thr Asp Phe Asp Phe Leu Lys Val Ile Gly Lys Gly Asn Tyr Gly Lys
   1               5                   10                  15
   Val Leu Leu Ala Lys Arg Lys Ser Asp Gly Ala Phe Tyr Ala Val Lys
               20                  25                  30
   Val Leu Gln Lys Lys Ser Ile Leu Lys Lys Lys Glu Gln Ser His Ile
           35                  40                  45
   Met Ala Glu Arg Ser Val Leu Leu Lys Asn Val Arg His Pro Phe Leu
       50                  55                  60
   Val Gly Leu Arg Tyr Ser Phe Gln Thr Pro Glu Lys Leu Tyr Phe Val
   65                  70                  75                  80
   Leu Asp Tyr Val Asn Gly Gly Glu Leu Phe Phe His Leu Gln Arg Glu
                   85                  90                  95
   Arg Arg Phe Leu Glu Pro Arg Ala Arg Phe Tyr Ala Ala Glu Val Ala
               100                 105                 110
   Ser Ala Ile Gly Tyr Leu His Ser Leu Asn Ile Ile Tyr Arg Asp Leu
           115                 120                 125
   Lys Pro Glu Asn Ile Leu Leu Asp Cys Gln Gly His Val Val Leu Thr
       130                 135                 140
   Asp Phe Gly Leu Cys Lys Glu Gly Val Glu Pro Glu Asp Thr Thr Ser
   145                 150                 155                 160
   Thr Phe Cys Gly Thr Pro Glu Tyr Leu Ala Pro Glu Val Leu Arg Lys
                   165                 170                 175
   Glu Pro Tyr Asp Arg Ala Val Asp Trp Trp Cys Leu Gly Ala Val Leu
               180                 185                 190
   Tyr Glu Met Leu His Gly Leu Pro Pro Phe Tyr Ser Gln Asp Val Ser
           195                 200                 205
   Gln Met Tyr Glu Asn Ile Leu His Gln Pro Leu Gln Ile Pro Gly Gly
       210                 215                 220
   Arg Thr Val Ala Ala Cys Asp Leu Leu Gln Ser Leu Leu His Lys Asp
   225                 230                 235                 240
   Gln Arg Gln Arg Leu Gly Ser Lys Ala Asp Phe Leu Glu Ile Lys Asn
                   245                 250                 255
   His Val Phe Phe
               260
<210> 12
   <211> 260
   <212> PRT
   <213> Homo sapiens
<400> 12
   Thr Asp Phe Asp Phe Leu Lys Val Ile Gly Lys Gly Ser Phe Gly Lys
   1               5                   10                  15
   Val Leu Leu Ala Lys Arg Lys Leu Asp Gly Lys Val Tyr Ala Val Lys
               20                  25                  30
   Val Leu Gln Lys Lys Ile Val Leu Asn Arg Lys Glu Gln Lys His Ile
           35                  40                  45
   Met Ala Glu Arg Asn Val Leu Leu Lys Asn Val Lys His Pro Phe Leu
       50                  55                  60
   Val Gly Leu His Tyr Ser Phe Gln Thr Thr Glu Lys Leu Tyr Phe Val
   65                  70                  75                  80
   Leu Asp Phe Val Asn Gly Gly Glu Leu Phe Phe His Leu Gln Arg Glu
                   85                  90                  95
   Arg Ser Phe Pro Glu His Arg Ala Arg Phe Tyr Ala Ala Glu Ile Ala
               100                 105                 110
   Ser Ala Leu Gly Tyr Leu His Ser Ile Lys Ile Val Tyr Arg Asp Leu
           115                 120                 125
   Lys Pro Glu Asn Ile Leu Val Asp Ser Val Gly His Val Val Leu Thr
       130                 135                 140
   Asp Phe Gly Leu Cys Lys Glu Gly Ile Ala Ile Ser Asp Thr Thr Thr
   145                 150                 155                 160
   Thr Phe Cys Gly Thr Pro Glu Tyr Leu Ala Pro Glu Val Ile Arg Lys
                   165                 170                 175
   Gln Pro Tyr Asp Asn Thr Val Asp Trp Trp Cys Leu Gly Ala Val Leu
               180                 185                 190
   Tyr Glu Met Leu Tyr Gly Leu Pro Pro Phe Tyr Cys Arg Asp Val Ala
           195                 200                 205
   Glu Met Tyr Asp Asn Ile Leu His Lys Pro Leu Ser Leu Arg Pro Gly
       210                 215                 220
   Val Ser Leu Arg Ala Trp Ser Ile Leu Glu Glu Leu Leu Glu Lys Asp
   225                 230                 235                 240
   Arg Gln Asn Arg Leu Gly Ala Lys Glu Asp Phe Leu Glu Ile Gln Asn
                   245                 250                 255
   His Pro Phe Phe
               260
<210> 13
   <211> 680
   <212> DNA
   <213> Mus musculus
<400> 13
   ggcacatcgt cctcactgac tttgggctct gcaaagagaa tattgagcat aacgggacaa 60
   catctacctt ctgtggcacg cctgagtatc tggctcctga ggtcctccat aaacagccgt 120
   atgaccggac ggtggactgg tggtgtcttg gggctgtcct gtatgagatg ctctacggcc 180
   tgcccccgtt ttatagccgg aacacggctg agatgtacga caatattctg aacaagcctc 240
   tccagttgaa accaaatatt acaaactcgg caaggcacct cctggaaggc ctcctgcaga 300
   aggaccggac caagaggctg ggtgccaagg atgactttat ggagattaag agtcatattt 360
   tcttctcttt aattaactgg gatgatctca tcaataagaa gattacaccc ccatttaacc 420
   caaatgtgag tgggcccagt gaccttcggc acttcgatcc cgagtttacc gaggagccgg 480
   tccccagctc catcggcagg tcccctgaca gcatccttgt cacggccagt gtgaaggaag 540
   cagcagaagc cttcctcggc ttctcctatg cacctcctgt ggattccttc ctctgagtgc 600
   tcccgggatg gttctgaagg acttcctcag cgtttcctaa agtgttttcg ttagcctttg 660
   gtggagttgc cagctgacag                                             680